# EUROPEAN PATENT APPLICATION

(11) **EP 4 193 994 A1**
(43) Date of publication of application: **14.06.2023**
(21) Application number: 21000346.3
(22) Date of filing: 08.12.2021
(51) Int. Cl.: A61K 31/49, A61K 31/502, A61P 11/00, A61P 31/14

(54) **COMBINATION OF 5-AMINO-2,3-DIHYDRO-1,4-PHTALAZINEDIONE AND A 6'-METHOXYCINCHONAN-9-OL FOR USE IN THE TREATMENT OF CORONAVIRAL INFECTIONS**

(71) Applicant: MetrioPharm AG, 8002 Zürich (CH)
(72) Inventor: The designation of the inventor has not yet been filed

(57) **Abstract**

The present application relates to a combination of 5-amino-2,3-dihydro-1,4-phthalazinedione or one of its pharmaceutically acceptable salts and a 6'-methoxycinchonan-9-ol or a pharmaceutically acceptable salt thereof for use in the prevention or treatment of coronaviral infections. In particular, the invention relates to a combination of 5-amino-2,3-dihydro-1,4-phthalazinedione sodium salt and quinine or quinidine for said purposes. Pharmaceutical compositions and advantageous formulation techniques are disclosed.

## Description

### FIIELD OF THE INVENTION

The present application relates to a combination of 5-amino-2,3-dihydro-1,4-phthalazinedione or one of its pharmaceutically acceptable salts and a 6'-methoxycinchonan-9-ol or a pharmaceutically acceptable salt thereof for use in the prevention or treatment of coronaviral infections. In particular, the invention relates to a combination of 5-amino-2,3-dihydro-1,4-phthalazinedione sodium salt and quinine or quinidine or pharmaceutically salt thereof for said purposes. Pharmaceutical compositions and advantageous formulation techniques are disclosed.

### BACKGROUND OF THE INVENTION

As a result of ecological, climatic and demographic changes, so-called 'emerging' viruses are increasingly being transmitted from their natural animal hosts to humans. Due to accelerated globalization, they bear the risk of triggering a pandemic. Emerging viruses may cause acute and often life-threatening diseases. *Coronaviridae* have become notorious for such transmissions. Examples are *Severe acute respiratory syndrome coronavirus* (SARS-CoV) and *Middle East respiratory syndrome-related coronavirus* (MERS-CoV), and most recently, the outbreak of *Severe acute respiratory syndrome coronavirus* 2 (SARS-CoV-2; COVID-19) in Wuhan, China. A total of over 244 million SARS-CoV-2 cases with about 4.9 million casualties worldwide have been reported by the Johns Hopkins University Coronavirus Resource Center, as of October 27^{th}, 2021. The incubation period of SARS-CoV-2 ranges between two days and two weeks, in some cases up to one month. The disease resulting from a SARS-CoV-2 infection is called COVID-19.

Typical symptoms of COVID-19 are fever, cough, and shortness of breath. However, the infection can also cause severe pulmonary injury, leading to rapid onset of progressive malfunction of the lungs, especially with regard to the ability to take up oxygen. This is usually associated with the malfunction of other organs. This acute lung injury (ALI) condition is associated with extensive lung inflammation and accumulation of fluid in the alveoli. It is characterized by diffuse pulmonary microvascular injury resulting in increased permeability and, thus, non-cardiogenic pulmonary edema. In consequence, this leads to pathologically low oxygen levels in the lungs. Other common symptoms associated with COVID-19 patients in ICU care are pulmonary embolism, thrombosis, venous thromboembolism and brain ischemia.

Coronaviruses are primarily spread through close contact, in particular through respiratory droplets from coughs and sneezes. In contrast to the SARS-CoV and MERS-CoV, SARS-CoV-2 can be transmitted from human to human during the incubation period while the infected patient does not show yet any symptoms of disease. Moreover, SARS-CoV-2 can already replicate in the throat. In contrast, the receptors for SARS -CoV and MERS-CoV are located deep in the lungs. Thus, SARS-CoV-2 can be transmitted much easier from human to human in comparison to SARS-CoV and MERS-CoV which strongly increases the infection rate.

In general, coronaviruses (family Coronaviridae, group Coronaviruses) form a relatively diverse group of large, enveloped, positive strand RNA viruses, which can cause different types of diarrhea and respiratory diseases in humans and animals. They have a very narrow host range and replicate very poorly in cell culture. However, cell culture systems for SARS-CoV-2 could be successfully established.

Sequencing of SARS-CoV-2 revealed an approx. 29.8 kbp genome consisting of 14 open reading frames. Moreover, the virus is phylogenetically closely related to the SARS-CoV (89.1% nucleotide similarity) (cf. Wu et al. (2020) Nature 579: 265-269). Like other coronaviruses, SARS-CoV-2 enters the cell by endocytosis and membrane fusion. The viruses are released from the cell by the secretory pathway. The natural reservoir of the virus is unknown.

To date, no specific therapeutic options for the treatment of SARS-CoV-2 infections, respectively COVID-19 are established. Some success could be achieved with the antiviral drugs remdesivir, favipiravir and molnupiravir. A nasal spray containing nanoantibodies against the SARS-CoV-2 spike protein is a promising development (AeroNabs). In severe stage COVID-19 patients the administration of the glucocorticoid dexamethasone showed to be effective.

Thus, there is a strong medical need for an effective pharmacological treatment for patients infected with SARS-CoV-2 or similar coronaviruses and for limiting the current epidemic spread of this virus. Ideally, such a pharmacological treatment should also offer at least a treatment option for future coronavirus outbreaks.

5-amino-2,3-dihydro-1,4-phthalazinedione sodium salt could dose-dependently inhibit the replication of SARS-CoV-2 *in vitro* (PCT/EP2021/000071). Such an effect was also described for 6'-methoxycinchonan-9-ols such as quinine and quinidine (WO 2021/219244).

Surprisingly, this effect could be significantly increased by the administration of a pharmaceutical combination of 5-amino-2,3-dihydro-1,4-phthalazinedione or one of its pharmaceutically acceptable salts or solvates, hydrates and a 6'-methoxycinchonan-9-ol or a pharmaceutically acceptable salt thereof. A combination of 5-amino-2,3-dihydro-1,4-phthalazinedione sodium salt with quinine sulfate or quinidine sulfate was able to inhibit SARS-CoV-2 replication *in vitro* at much lower concentrations than the single substances.

Thus, the present application discloses a pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof.

Especially, a pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof for use in medicine is disclosed.

In particular a pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof for use in the prophylaxis or treatment of a coronaviral infection is disclosed, in particular in the prophylaxis or treatment of a SARS-CoV-2 infection, respectively COVID-19.

### DESCRIPTION OF THE INVENTION

5-amino-2,3-dihydro-1,4-phthalazinedione (luminol) belongs to the pharmaceutical class of the phthalazinediones. Compounds of this class are known for their beneficial antiinflammatory action. 5-amino-2,3-dihydro-1,4-phthalazinedione is also known under the name luminol. Luminol has excellent chemiluminescent properties. It is widely applied in diagnostic assays as a detection means and in forensic medicine, for example for tracing blood spots. In medicine, 5-amino-2,3-dihydro-1,4-phthalazinedione has been developed in the form of a sodium salt. In some countries it is approved for a broad range of acute and chronic inflammatory disorders, including i.a. acute infections of bacterial and viral origin, particularly of the intestinal tract, hepatitis B and C, gastroenteritis, inflammations such as prostatitis, endometriosis, throat inflammation, bronchial asthma, pneumonia, periodontitis, pyelonephritis and autoimmune diseases such as Crohn's disease, ulcerative colitis, lupus erythematosus and scleroderma. 5-amino-2,3-dihydro-1,4-phthalazinedione could effectively prevent cytokine storms caused by excessive immune reactions. Further, there is still a long list of indications in scientific and patent literature in the treatment of which 5-amino-2,3-dihydro-1,4-phthalazinedione sodium salt was allegedly tested or a beneficial use was suggested (cf. WO 2004/041169; WO 2007/018546; WO 2012/127441; WO 2016/096143; WO 2017/202496; WO 2018/082814: i.a.).

While most conventional immunomodulatory drugs show serious adverse reactions, or are at least problematic in long-term treatment, 5-amino-2,3-dihydro-1,4-phthalazinedioneand its pharmaceutically acceptable salts are well tolerated and have a high safety margin in respect to administered dosages.

To ensure a better solubility and bioavailability pharmaceutically acceptable salts of 5-amino-2,3-dihydro-1,4-phthalazinedione are used. Sodium, potassium and lithium salts have been described for therapeutic applications (cf. WO 2010/082858). Crystal structures for lithium, sodium, potassium, rubidium and cesium salts were described in Guzei et al. (2013) Journal of Coordination Chemistry 66, 3722-3739. Thus, the present patent application refers also to the use of all pharmaceutically acceptable salts of 5-amino-2,3-dihydro-1,4-phthalazinedione.

There are two diastereomers of 6'-methoxycinchonan-9-ol: Quinine ((-)-(8*α*,9*R*)-6'-methoxycinchonan-9-ol; synonyms: 1-(6-methoxyquinoline-4-yl)-1-(5-vinyl-1-azabicyclo[2.2.2]oct-2-yl)methanol, 1-(6-methoxyquinoline-4-yl)-1-(5-vinyl-1,4-ethanopiperidine-2-yl)methanol ) and quinidine ((+)-(9*S*)-6'-methoxycinchonan-9-ol; synonym: (2-ethenyl-4-azabicyclo[2.2.2]oct-5-yl)-(6-methoxyquinoline-4-yl)-methanol).

Thus, in the scope of the present disclosure the terms "6'-methoxycinchonan-9-ols" and "a 6'-methoxycinchonan-9-ol" shall refer to quinine and/or quinidine or a pharmaceutically acceptable salt thereof, if not expressly referenced otherwise.

Quinine and quinidine are quinoline alkaloids. Traditionally, quinine was obtained from quina bark *(Cinchona pubescens,* a rubiacea growing in high altitude forests in South America).

Quinine is a white, poorly water-soluble crystalline powder with a bitter taste. It was used long time in folk medicine for treating febrifugal diseases. Quinine was the first drug found to be effective in the treatment of malaria, in particular of the complicated form Malaria tropica. It complexes toxic ferriprotoporphyrin IX and thus inhibits the formation of non-toxic betahematin in the vacuoles of blood schizonts of *Plasmodium falciparum.* Nowadays it is still in use in the treatment of chloroquine-refractory malaria pathogens and if artemisinins are not indicated. In the USA it is often used against Malaria tropica. It is further used against babesiosis *(Babesia* infections).

Quinine has also analgesic, local anesthetic and antipyretic properties. Therefore, low doses of quinine are used in China for treating common colds.

When administered in low doses as quinine sulfate it also used to resolve cramps such as leg cramps at night.

It has also been in use for treating restless leg syndrome but was discontinued because of side effects.

Further indications are lupus erythematosus, osteoarthrosis and rheumatoid arthritis.

Adverse side effects include thrombocytopenia, thrombotic microangiopathy and methemoglobinemia. Long-term therapy with high doses of quinine sulfate may lead to nausea, headache, sweating, tinnitus, visual disturbances, fever, hypotension, hemolytic anemia, acute kidney injury, liver toxicity, blindness and disorders of the gastrointestinal tract, the dermis, the cardiovascular system (low platelet count, hemolytic-uremic syndrome/thrombotic thrombocytopenic purpura (HUS/TTP), long QT syndrome and other serious cardiac arrhythmias including torsades de pointes, blackwater fever, disseminated intravascular coagulation, leukopenia, neutropenia) and the nervous system, in rare cases asthma, hemoglobinemia (cf. Liles et al. (2016) Am J Hematol 91: 461-466).

In the food industry, quinine is used as a bitter flavoring. It can be added to fancy drinks like bitter lemon or tonic water (in the European Union up to 100 mg/kg, in Germany 85 mg/kg) or to alcoholic beverages like gin tonic and bitters (in the European Union up to 300 mg/kg, in Germany 250 mg/kg).

Quinidine was the first antiarrhythmic agent that was found to be effective. It can be administered intravenously or orally. It is classified as a class 1A antiarrhythmic, as it binds to open sodium channels (in particular Naᵥ1.5). There is frequency-dependency of its action (use-dependent block), as the quinidine / ion channel complex only slowly dissociates. Quinidine also decreases the potassium conductivity (in particular Kᵥ1.4, Kᵥ4.2, Kᵥ11.1), leading to increased cardiac action potential duration and a prolonged QT interval. Moreover, quinidine blocks cardiac calcium channels in an atropine-like manner and is an alpha-1 blocker (Shibata et al. (1998) Circulation 97: 1227-1230). Quinidine is thus used in the treatment of atrial fibrillation, extrasystoles and ventricular tachyarrhythmias.

Seldomly, intravenously administered quinidine is used against *Plasmodium falciparum* malaria.

It is nowadays seldomly used because of serious adverse side effects. These effects include long QT syndrome, atrioventricular block, torsade de pointes arrhythmias, ventricular tachycardias as well as gastrointestinal disorders). When administered intravenously, quinidine strongly reduces vascular resistance. Further adverse side effects include thrombocytopenia (eventually leading to thrombocytic purpura), granulomatous hepatitis and myasthenia gravis.

Further, quinidine inhibits cytochrome P450 enzyme 2D6 and thus increases the blood level of a number of medications.

Quinine and quinidine can be provided as pharmaceutically acceptable salts of organic and inorganic acids. Suitable examples are hydrochloric acid, hydrobromic acid, sulfuric acid, phosphoric acid, acetic acid, citric acid, oxalic acid, malonic acid, salicylic acid, p-aminosalicylic acid, malic acid, fumaric acid, succinic acid, ascorbic acid, maleic acid, sulfonic acid, phosphonic acid, perchloric acid, nitric acid, formic acid, propionic acid, gluconic acid, digluconic acid, lactic acid, tartaric acid, hydroxymaleic acid, pyruvic acid, phenylacetic acid, benzoic acid, p-aminobenzoic acid, p-hydroxybenzoic acid, dinitrobenzoic acid, chlorbenzoic acid, methanesulfonic acid, ethanesulfonic acid, nitric acid, hydroxyethanesulfonic acid, ethylenesulfonic acid, p-toluylsulfonic acid, naphthylsulfonic acid, sulfanilic acid, camphorsulfonic acid, alginic acid, capric acid, hippuric acid, pectinic acid, phthalic acid, quinic acid, mandelic acid, o-methyl mandelic acid, hydrogen benzenesulfonic acid, picric acid, adipic acid, cyclopentane propionic acid, D-o-toluyl tartaric acid, tartronic acid, benzenesulfonic acid, alpha-methyl benzoic acid, (o, m, p-)methyl benzoic acid, naphthylamine sulfonic acid, as well as salts from other mineral acids or carbonic acids well known to a person skilled in the art. These salts are generated by contacting the free base with a sufficient amount of the respective acid in order to build the salt in a conventional manner.

Frequently used salts of quinine include quinine sulfate, quinine dihydrochloride, quinine gluconate, quinine hydrochloride and quinine sulfate dihydrate. Preferred are quinine sulfate and quinine dihydrochloride. Frequently used salts of quinidine include quinidine sulfate, quinidine dihydrochloride, quinidine gluconate, quinidine hydrochloride monohydrate, quinidine citrate, quinidine acetate and quinidine sulfate dihydrate. Preferred are quinidine sulfate and quinidine gluconate. Further salts of quinine and quinidine are revealed in US 2009/239900 A1.

Pharmaceutically acceptable salts should be seen in terms of this application as an active agent containing a compound according to the invention in form of a salt, in particular if this salt bestows specific or ameliorated pharmacokinetic properties in comparison to the free form of the active agent or to another salt of the active agent. The pharmaceutically acceptable salt of the active agent may also bestow a pharmacokinetic characteristic to the active agent it did not have in its free form. Thus, it may even positively influence the pharmacodynamics of the active agent in respect to its therapeutic efficacy in the organism.

It is therefore desirable to find a medication that allows for boosting the effects of quinine and/or quinidine or pharmaceutically salt thereof in coronaviral infections for increasing the therapeutic efficacy. A further desirable action would be that the administered dosage of the respective antiviral could be reduced for avoiding or at least mitigating adverse side effects associated with them.

To avoid ambiguities in the art, the following terms of interaction among drugs are defined. They are used in this sense throughout the disclosure. If at least two substances (e.g., pharmaceutical agents) are administered and at least one of these substances affects the activity of the other at least one substance a so-called drug-drug interaction is given. If said interaction leads to an exaggerated or increased effect of the drug substance the effect is synergistic. The respective equation can be displayed as (A+B) > A or (A+B) > B, wherein A and B are the percentual or fractional effects (i.e., a value between 0 and 1) seen when the respective substances are administered alone and (A+B) is the percentual or fractional effect seen after combinatory application, respectively. A final effect equal to the sum of the effects seen in the single drug applications is an additive synergistic effect. If the final effect is greater than said expected effect the term supraadditivity is used. Smaller effects are subadditive. If, on the other hand the drug-drug interaction leads to a decreased effect of the drug substance the effect is not synergistic at all, but antagonistic.

Hence, in the scope of this disclosure the terms "antagonism" and "antagonistic" are used for any decreased drug effect induced by drug-drug-interaction, whilst the terms "synergy" and "synergistic" are used for any increased drug effect induced by drug-drug-interaction. To describe and determine the grade of said synergy the terms "subadditive", "additive" and "supraadditive" as well as the respective nouns are used. Hence, the term supraadditivity is used to describe an effect of two or more combined agents that is greater than the expected additive effects of the single agents.

The use of synergistic acting drug combinations can both help to increase the therapeutic efficacy and the potency, wherein the latter primarily helps to reduce off-target toxicity.

The identification of drug-drug-interactions depends on the "no-interaction" null-hypotheses, which is based on the observed drug response and not on any model of mechanism. Hence, for two different drugs, their grade of additivity is based on a point of reference for the readout wherein the point of reference depends on the mathematical model chosen. Various methods exist to identify such interactions and to calculate the expected additive effect of two substances and thus to determine if the actual synergistic effect observed is subadditive, additive or supraadditive. Different methods are recommended in the art. The most common methods are outlined below.

Basic methods like building simple arithmetic sums or the fractional product method provide an easy way to gain first insights if a specific combination has supraadditive potential.

The simple arithmetic sum method for additivity is based on the equation A + B = (A+B), wherein A and B again are the percentual or fractional effects seen when the respective substances are administered alone and (A+B) is the percentual or fractional effect seen after combinatory application of said substances using the same doses as in the single applications. If A + B > (A+B) supraadditivity is given. An obviously striking disadvantage of this method is that results wherein A + B ≥ 100% cannot be analyzed for supraadditivity. Hence, this method is primarily used to interpret combinations of suboptimal doses.

The fractional product method is based on the equation 1 - (1-A)_{*}(1-B) = (A+B). Here, A and B are the fractual effects seen when the respective substances are administered alone and (A+B) is the fractual effect seen after combinatory application of said substances using the same doses as in the single applications. If 1 - (1-A)_{*}(1-B) > (A+B), supraadditivity is given. More sophisticated methods include the use of a so-called isobologram, a graph constructed on a coordinate system defined by individual drug doses showing a "line of additivity" that allows to distinguish between subadditive, additive (along the line) and supraadditive effects. The "line of additivity" connects those single drug doses which display the same effect (e.g., 50% inhibition of a specific marker). All possible dose combinations along this line are expected to show the same effectiveness. Dose combinations located within the triangle built by coordinates and the line of additivity i.e., nearer to the arbitrary point, displaying the same effect are considered as supraadditive. Dose combinations located outside the triangle are considered as subadditive. Respective figures can be mapped using specific software as e.g., CompuSyn (Chou and Martin, ComboSyn, Inc. Paramus, NJ 2007 [www.combosyn.com]).

Also, further specific indicator values as for example the Combination Index (CI, equation of Chou and Talalay (1984) Adv Enzyme Reg 22: 27-55) and the Dose-Reduction Index (DRI, Chou equation 1984) can be calculated easily using the beforementioned specific software. Both these values allow to determine if drug substances act supraadditive synergistically when administered contemporarily. The CI is based on the principles of mass action law and is applicable for any kind of drug combination regardless of mechanism of action, dynamic order or the quantity units used for each drug in the combination. The CI value defines synergism as supraadditive if CI<1 and as additive if CI =1. If CI > 1 the effect is either subadditive or antagonistic. Hence, a CI of 1 also refers to the "line of additivity" In the classic isobologram as mentioned above. In a simplified approach it could be calculated as follows: CI = A(t)/A(x) + B(t)/B(x), wherein A(t) respectively B(t) is the dose of drug A respectively B alone that inhibits x %, whereas A(x) and B(x) stand for the portion of the respective drug in the combination that also inhibits x %. The DRI is a measure of how many folds the dose of each drug in a synergistic combination may be reduced at a given effect level compared with the doses of each drug alone. Therefore, DRI=1 indicates additivity, whereas DRI>1 and <1 indicate supraadditivity and subadditivity (or antagonism), respectively. For displaying purposes, the isobologram, i.e., the equi-effective curve at various concentrations or doses of two drugs as mentioned above, is a dose-oriented figure approach based on a special case of the CI equation. A more convenient figure approach, however, is the effect oriented so-called FaCI plot, displaying the Combination Index (CI) against the fractual effect (Fa), preferably for a specific dose combination.

In another aspect the present application refers to a pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof for use in the reduction or inhibition of the replication of a coronavirus in a human.

In another aspect the present application refers to a pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof for use in the reduction of the viral load of a coronavirus in a human.

In another aspect the present application refers to a pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof for use in the prophylaxis or treatment of COVID-19 in a human.

The severity of a COVID-19 is usually classified according to the WHO Clinical Progression Scale (published in: Lancet Infect Dis (2020) 20: e192-e197). In the scope of the present disclosure the following classifications follow this scale.

In another aspect the present application refers to a pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof for use in the prophylaxis or treatment of a coronaviral infection in a human, wherein said human is asymptomatic.

In another aspect the present application refers to a pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof for use in the prophylaxis or treatment of a coronaviral infection in a human, wherein said human shows mild coronavirus infection- related symptoms and is not in need of hospitalization.

In another aspect the present application refers to a pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof for use in the treatment of a coronaviral infection in a human, wherein said human shows severe coronavirus infection-related symptoms and is in need of hospitalization.

In another aspect the present application refers to a pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof for use in the treatment of a coronaviral infection in a human, wherein said human shows severe coronavirus infection-related symptoms and is undergoing acute lung injury.

In particular, the present application discloses a pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof for use in the prophylaxis or treatment of a coronaviral infection, wherein the pharmaceutically acceptable salt of 5-amino-2,3-dihydro-1,4-phthalazinedione is 5-amino-2,3-dihydro-1,4-phthalazinedione sodium salt.

In another aspect the present application refers to a pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof for use in the reduction or inhibition of the replication of a coronavirus in a human, wherein the pharmaceutically acceptable salt of 5-amino-2,3-dihydro-1,4-phthalazinedione is 5-amino-2,3-dihydro-1,4-phthalazinedione sodium salt.

In another aspect the present application refers to a pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof for use in the reduction of the viral load of a coronavirus in a human, wherein the pharmaceutically acceptable salt of 5-amino-2,3-dihydro-1,4-phthalazinedione is 5-amino-2,3-dihydro-1,4-phthalazinedione sodium salt.

In another aspect the present application refers to a pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof for use in the prophylaxis or treatment of COVID-19 in a human, wherein the pharmaceutically acceptable salt of 5-amino-2,3-dihydro-1,4-phthalazinedione is 5-amino-2,3-dihydro-1,4-phthalazinedione sodium salt.

In another aspect the present application refers to a pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof for use in the prophylaxis or treatment of a coronaviral infection in a human, wherein the pharmaceutically acceptable salt of 5-amino-2,3-dihydro-1,4-phthalazinedione is 5-amino-2,3-dihydro-1,4-phthalazinedione sodium salt and said human is asymptomatic.

In another aspect the present application refers to a pharmaceutical combination of 5-amino-2,3-dihydro-1,4-phthalazinedione or one of its pharmaceutically acceptable salts, hydrates or solvates and at a 6'-methoxycinchonan-9-ol or a pharmaceutically acceptable salt thereof for use in the prophylaxis or treatment of a coronaviral infection in a human, wherein the pharmaceutically acceptable salt of 5-amino-2,3-dihydro-1,4-phthalazinedione is 5-amino-2,3-dihydro-1,4-phthalazinedione sodium salt and said human shows mild coronavirus-infection related symptoms and is not in need of hospitalization.

In another aspect the present application refers to a pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof for use in the treatment of a coronaviral infection in a human, wherein the pharmaceutically acceptable salt of 5-amino-2,3-dihydro-1,4-phthalazinedione is 5-amino-2,3-dihydro-1,4-phthalazinedione sodium salt and said human shows severe coronavirus-infection related symptoms and is in need of hospitalization.

In another aspect the present application refers to a pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof for use in the treatment of a coronaviral infection in a human, wherein the pharmaceutically acceptable salt of 5-amino-2,3-dihydro-1,4-phthalazinedione is 5-amino-2,3-dihydro-1,4-phthalazinedione sodium salt and said human shows severe coronavirus-infection related symptoms and is undergoing acute lung injury.

5-amino-2,3-dihydro-1,4-phthalazinedione is often used as a hydrate, for example as sodium salt dihydrate. Thus, the present patent application refers also to the use of all hydrates and other solvates of 5-amino-2,3-dihydro-1,4-phthalazinedione and its pharmaceutically acceptable salts. 5-amino-2,3-dihydro-1,4-phthalazinedione or one of its pharmaceutically acceptable salts may build complexes with suitable ligands. Thus, the present patent application refers also to such complexes.

For ensuring a reproducible and standardized API production and to provide improved stability features of an active agent anhydrous formulations are often preferred. Anhydrate forms of 5-amino-2,3-dihydro-1,4-phthalazinedione sodium salt have been described as crystalline polymorphs in WO 2011/107295 (Form I, Form II) and WO 2016/096143 (Form III). These crystalline polymorphs are virtually free from phase impurities and were characterized by means of X-ray powder diffraction. This method yields a set of characteristic d-values indicating interplanar spacings [Å] and of the corresponding 2-theta (2θ) angles [°] under which Bragg reflections occur. This yields a unique and unambiguous fingerprint of the respective polymorphs.

For Form I the following values were determined:
d values: 13.5; 6.9; 5.2; 4.6; 3.9; 3.5; 3.4; 3.3; 3.1; 3.0 and/or
2-theta values: 6.5; 12.7; 16.9; 19.3; 22.8; 25.8; 26.6; 27.2; 28.7; 30.3.

Form II is characterized by the following values:
d values: 12.9; 7.9; 7.1; 6.5; 5.3; 4.0; 3.7; 3.6; 3.3; 3.2 and/or
2-theta values: 6.8; 11.2; 12.5; 13.7; 16.7; 22.4; 24.3; 24.9; 27.2; 27.8.

Form III yielded the following values:
d values: 13.131; 7.987; 7.186; 6.566; 6.512; 5.372; 3.994; 3.662; 3.406; 3.288; 3.283; 3.222; 3.215; 3.127; 2.889 and/or
2-theta values: 6.73; 11.07; 12.31; 13.48; 13.59; 16.49; 22.24; 24.29; 26.14; 27.10; 27.14; 27.67; 27.72; 28.52; 30.93.

The use of anhydrous Form I of 5-amino-2,3-dihydro-1,4-phthalazinedione sodium salt is preferred.

In a murine COPD model, an ex *vivo* lung was exposed to cigarette smoke. The application of 5-amino-2,3-dihydro-1,4-phthalazinedione sodium salt Form I led to a nearly complete reduction of 3-nitrotyrosine. Therefore it is assumed that 5-amino-2,3-dihydro-1,4-phthalazinedione sodium salt may protect the lung from oxidative and nitrosative stress which are major players in the pathophysiology of COPD and other inflammatory pulmonary diseases (https://copdnewstoday.com/2020/04/16/mp1032-may-protect-lungs-from-oxidative-stress-inhibit-biomarker-3-nitrotyrosine-preclinical-study/ , as of 2021-03-03).

5-amino-2,3-dihydro-1,4-phthalazinedione itself shows also polymorphism. A Form I (Paradies (1992) Ber. Bunsen-Ges. Phys. Chem 96: 1027-1031) and a Form II (WO 2017/140430) have been disclosed.

WO 2017/140430 discloses also that 5-amino-2,3-dihydro-1,4-phthalazinedione has a great potential in the immunomodulatory treatment of inflammatory and autoimmune diseases. Crystalline Form II is particularly useful in the treatment of inflammatory and autoimmune respiratory diseases such as upper and lower respiratory tract infections.

Thus, the present patent application refers also to the use according to the invention of all crystalline forms and polymorphs thereof of 5-amino-2,3-dihydro-1,4-phthalazinedione and its pharmaceutically acceptable salts, hydrates and solvates. The use of Form II of 5-amino-2,3-dihydro-1,4-phthalazinedione is preferred.

Similar therapeutic effects are known for a variety of phthalazinediones, respectively of derivatives of 5-amino-2,3-dihydro-1,4-phthalazinedione and its pharmaceutically acceptable salts. An example is 6-amino-2,3-dihydrophthalazine-1,4-dione (isoluminol). An overview of suitable phthalazinediones is given in WO 2007/018546. It is reasonable to assume that these compounds show comparable effects when being used for the therapeutic applications according to the invention.

In EP 0 531 370 A1 the use of a PARP (poly(ADP-ribose) polymerase) inhibitor such as luminol is disclosed for the treatment of a viral infection in which the virus DNA is integrated into a host chromosome during the replication cycle. The virus is preferably a retrovirus such as HIV. Coronaviruses, however, are positive strand RNA viruses and therefore cannot integrate into the human DNA. Therefore EP 0 531 370 A1 does not suggest the use of 5-amino-2,3-dihydro-1,4-phthalazinedione or one of its pharmaceutically acceptable salts for the use in the treatment of a coronaviral infection.

Tautomerism relates to a rapid intraconversion of organic compounds in which a hydrogen atom or proton formally migrates inside the compound. This is accompanied by a switch of a single bond and adjacent double bond. The single forms are called tautomers. For example, keto-enol tautomerism occurs in 5-amino-2,3-dihydro-1,4-phthalazinedione (Proescher and Moody (1939) J Lab Clin Med, 1183-1189). Thus, the present patent application refers also to the use of all tautomers of 5-amino-2,3-dihydro-1,4-phthalazinedione and its pharmaceutically acceptable salts, hydrates and solvates.

Isomer is a generic term for molecules with the same chemical formula but a different chemical structure. They can be differentiated into constitutional (structural) isomers (wherein an exchange of atoms or of a functional group occurs) and stereoisomers. Stereoisomers can be subdivided into enantiomers (non-superimposable mirror images of the same molecule) and diastereomers (the same molecule with a different configuration at one or more stereocenters). Diastereomers can be subdivided into cis/trans isomers (referring to the relative orientation of functional groups within a molecule) and on the other hand conformers (rotation about formally single bonds) and rotamers (different rotational positioning about a single bond). An example for a constitutional isomer of 5-amino-2,3-dihydro-1,4-phthalazinedione is 6-amino-2,3-dihydrophthalazine-1,4-dione (isoluminol). Stereoisomers may occur in phthalazinedione derivatives. Thus, the present patent application refers also to the use of all isomers of 5-amino-2,3-dihydro-1,4-phthalazinedione, its derivatives and pharmaceutically acceptable salts, hydrates and solvates.

For some applications it may be desirable that isotopically enriched forms of the compounds of the invention are used e.g., for diagnostic purposes. Thus, the present patent application refers also to such isotopically enriched forms of the compounds of the invention.

From a pharmacokinetic point of view or for a production rationale it may be preferable to use a prodrug as a dosage form. A prodrug is administered in a pharmacologically inactive form and is metabolically converted into the active form inside the body. This conversion may occur systemically or locally. Thus, the present patent application refers also to prodrugs of the compounds of the invention.

As used throughout the present application the term "5-amino-2,3-dihydro-1,4-phthalazinedione or one of its pharmaceutically acceptable salts" shall encompass all the beforementioned molecular variants of 5-amino-2,3-dihydro-1,4-phthalazinedione i.e., 5-amino-2,3-dihydro-1,4-phthalazinedione or one of its pharmaceutically acceptable salts or solvates, hydrates, crystalline polymorphs, tautomers or isotopically enriched forms thereof.

Unless otherwise stated, any technical or scientific term used in the present invention has the meaning that a man skilled in the relevant technical art will attribute to them.

Coronaviral infections that can be treated with a pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof are above all infections with the highly pathogenic SARS-CoV, MERS-CoV and SARS-CoV-2. But also infections with less pathogenic coronaviridae as listed in the following can be thus treated. The terms "coronavirus" or "coronaviral" refer mainly to the sub-family of orthocoronavirinae. They are subdivided into the genera of alphacoronaviruses, betacoronaviruses, gammacoronaviruses and deltacoronaviruses. Alphacoronaviruses comprise the sub-genera of colacoviruses (species: bat coronavirus CDPHE15), decaviruses (bat coronavirus HKU10, Rhinolophus ferrumequinum alphacoronavirus HuB-2013), duvinacoviruses (human coronavirus 229E), luchacoviruses (Lucheng Rn rat coronavirus), minacoviruses (Ferret coronavirus, Mink coronavirus 1), minunacoviruses (miniopterus bat coronavirus 1, miniopterus bat coronavirus HKU8), myotacoviruses (Myotis ricketti alphacoronavirus Sax-2011), nylactoviruses (Nyctalus velutinus alphacoronavirus SC-2013), pedacoviruses (porcine epidemic diarrhea virus, Scotophilus bat coronavirus 512), rhinacoviruses (Rhinolophus bat coronavirus HKU2), setracoviruses (human coronavirus NL63, NL63-related bat coronavirus strain BtKYNM63-9b) and tegacoviruses (Alphacoronavirus 1 - type species). Betacoronaviruses comprise the sub-genera of embecoviruses (Betacoronavirus 1 (subspecies: human coronavirus OC43), China Rattus coronavirus HKU24, human coronavirus HKU1, murine coronavirus-type species), hibecoviruses (Bat Hp-betacoronavirus Zhejiang 2013), merbecoviruses (Hedgehog coronavirus 1, MERS-CoV), Pipistrellus bat coronavirus HKU5, Tylonycteris bat coronavirus HKU4), nobecoviruses (Rousettus bat coronavirus GCCDC1, Rousettus bat coronavirus HKU9 and sarbecoviruses (severe acute respiratory syndrome-related coronavirus (subspecies: SARS-CoV, SARS-CoV-2). Gammacoronaviruses comprise the sub-genera of cegacoviruses (Beluga whale coronavirus SW1) and igacoviruses (Avian coronavirus-type species). Deltacoronaviruses comprise the sub-genera of andecoviruses (Wigeon coronavirus HKU20), buldecoviruses (Bulbul coronavirus HKU11-type species, Porcine coronavirus HKU15, Munia coronavirus HKU13, White-eye coronavirus HKU16), herdecoviruses (Night heron coronavirus HKU19) and Moordecoviruses (Common moorhen coronavirus HKU21).

Coronaviruses pathogenic in humans are until now SARS-CoV, SARS-CoV-2, MERS-CoV and HCoV-HKU1, HCoV-NL-63, HCoV-OC43 and HCoV-229E. The last four cause only relatively mild symptoms (cf. Andersen et al.: The Proximal Origin of SARS-CoV-2, on virologica.org, as of February 17^{th}, 2020).

Thus, the present application relates particularly to a pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof for use in the prophylaxis or treatment of a coronaviral infection, wherein said coronaviral infection is selected from a group consisting of SARS-CoV, SARS-CoV-2, MERS-CoV, HCoV-HKU1, HCoV-NL-63, HCoV-OC43 and HCoV-229E infections.

In another aspect the present application refers to a pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof for use in the reduction or inhibition of the replication of a coronavirus in a human, wherein said coronaviral infection is selected from a group consisting of SARS-CoV, SARS-CoV-2, MERS-CoV, HCoV-HKU1, HCoV-NL-63, HCoV-OC43 and HCoV-229E infections.

In another aspect the present application refers to a pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof for use in the reduction of the viral load of a coronavirus in a human, wherein said coronaviral infection is selected from a group consisting of SARS-CoV, SARS-CoV-2, MERS-CoV, HCoV-HKU1, HCoV-NL-63, HCoV-OC43 and HCoV-229E infections.

In another aspect the present application refers to a pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof for use in the prophylaxis or treatment of a coronaviral infection in a human, wherein said human is asymptomatic and said coronaviral infection is selected from a group consisting of SARS-CoV, SARS-CoV-2, MERS-CoV, HCoV-HKU1, HCoV-NL-63, HCoV-OC43 and HCoV-229E infections.

In another aspect the present application refers to a pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof for use in the prophylaxis or treatment of a coronaviral infection in a human, wherein said human shows mild coronavirus infection- related symptoms, is not in need of hospitalization and said coronaviral infection is selected from a group consisting of SARS-CoV, SARS-CoV-2, MERS-CoV, HCoV-HKU1, HCoV-NL-63, HCoV-OC43 and HCoV-229E infections.

In another aspect the present application refers to a pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof for use in the treatment of a coronaviral infection in a human, wherein said human shows severe coronavirus infection-related symptoms, is in need of hospitalization and said coronaviral infection is selected from a group consisting of SARS-CoV, SARS-CoV-2, MERS-CoV, HCoV-HKU1, HCoV-NL-63, HCoV-OC43 and HCoV-229E infections.

In another aspect the present application refers to a pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof for use in the treatment of a coronaviral infection in a human, wherein said human shows severe coronavirus infection-related symptoms, is undergoing acute lung injury and said coronaviral infection is selected from a group consisting of SARS-CoV, SARS-CoV-2, MERS-CoV, HCoV-HKU1, HCoV-NL-63, HCoV-OC43 and HCoV-229E infections.

In another aspect the present application refers to a pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof for use in the reduction or inhibition of the replication of a coronavirus in a human, wherein the pharmaceutically acceptable salt of 5-amino-2,3-dihydro-1,4-phthalazinedione is 5-amino-2,3-dihydro-1,4-phthalazinedione sodium salt and said coronaviral infection is selected from a group consisting of SARS-CoV, SARS-CoV-2, MERS-CoV, HCoV-HKU1, HCoV-NL-63, HCoV-OC43 and HCoV-229E infections.

In another aspect the present application refers to a pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof for use in the reduction of the viral load of a coronavirus in a human, wherein the pharmaceutically acceptable salt of 5-amino-2,3-dihydro-1,4-phthalazinedione is 5-amino-2,3-dihydro-1,4-phthalazinedione sodium salt and said coronaviral infection is selected from a group consisting of SARS-CoV, SARS-CoV-2, MERS-CoV, HCoV-HKU1, HCoV-NL-63, HCoV-OC43 and HCoV-229E infections..

In another aspect the present application refers to a pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof for use in the prophylaxis or treatment of a coronaviral infection in a human, wherein the pharmaceutically acceptable salt of 5-amino-2,3-dihydro-1,4-phthalazinedione is 5-amino-2,3-dihydro-1,4-phthalazinedione sodium salt and said human is asymptomatic and said coronaviral infection is selected from a group consisting of SARS-CoV, SARS-CoV-2, MERS-CoV, HCoV-HKU1, HCoV-NL-63, HCoV-OC43 and HCoV-229E infections.

In another aspect the present application refers to a pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof for use in the prophylaxis or treatment of a coronaviral infection in a human, wherein the pharmaceutically acceptable salt of 5-amino-2,3-dihydro-1,4-phthalazinedione is 5-amino-2,3-dihydro-1,4-phthalazinedione sodium salt and said human shows mild coronavirus-infection related symptoms, is not in need of hospitalization and said coronaviral infection is selected from a group consisting of SARS-CoV, SARS-CoV-2, MERS-CoV, HCoV-HKU1, HCoV-NL-63, HCoV-OC43 and HCoV-229E infections.

In another aspect the present application refers to a pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof for use in the treatment of a coronaviral infection in a human, wherein the pharmaceutically acceptable salt of 5-amino-2,3-dihydro-1,4-phthalazinedione is 5-amino-2,3-dihydro-1,4-phthalazinedione sodium salt and said human shows severe coronavirus-infection related symptoms, is in need of hospitalization and said coronaviral infection is selected from a group consisting of SARS-CoV, SARS-CoV-2, MERS-CoV, HCoV-HKU1, HCoV-NL-63, HCoV-OC43 and HCoV-229E infections.

In another aspect the present application refers to a pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof for use in the treatment of a coronaviral infection in a human, wherein the pharmaceutically acceptable salt of 5-amino-2,3-dihydro-1,4-phthalazinedione is 5-amino-2,3-dihydro-1,4-phthalazinedione sodium salt and said human shows severe coronavirus-infection related symptoms, is undergoing acute lung injury and said coronaviral infection is selected from a group consisting of SARS-CoV, SARS-CoV-2, MERS-CoV, HCoV-HKU1, HCoV-NL-63, HCoV-OC43 and HCoV-229E infections.

Thus the present application relates in particular to a pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof for use in the prophylaxis or treatment of a coronaviral infection, wherein said coronaviral infection is a SARS-CoV-2 infection.

In another aspect the present application refers to a pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof for use in the reduction or inhibition of the replication of a coronavirus in a human, wherein said coronaviral infection is a SARS-CoV-2 infection.

In another aspect the present application refers to a pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof for use in the reduction of the viral load of a coronavirus in a human, wherein said coronaviral infection is a SARS-CoV-2 infection.

In another aspect the present application refers to a pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof for use in the prophylaxis or treatment of a coronaviral infection in a human, wherein said human is asymptomatic and said coronaviral infection is a SARS-CoV-2 infection.

In another aspect the present application refers to a pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof for use in the prophylaxis or treatment of a coronaviral infection in a human, wherein said human shows mild coronavirus-infection related symptoms, is not in need of hospitalization and said coronaviral infection is a SARS-CoV-2 infection.

In another aspect the present application refers to a pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof for use in the treatment of a coronaviral infection in a human, wherein said human shows severe coronavirus-infection related symptoms, is in need of hospitalization and said coronaviral infection is a SARS-CoV-2 infection.

In another aspect the present application refers to a pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof for use in the treatment of a coronaviral infection in a human, wherein said human shows severe coronavirus-infection related symptoms, is undergoing acute lung injury and said coronaviral infection is a SARS-CoV-2 infection.

In another aspect the present application refers to a pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof for use in the reduction or inhibition of the replication of a coronavirus in a human, wherein the pharmaceutically acceptable salt of 5-amino-2,3-dihydro-1,4-phthalazinedione is 5-amino-2,3-dihydro-1,4-phthalazinedione sodium salt and said coronaviral infection is a SARS-CoV-2 infection.

In another aspect the present application refers to a pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof for use in the reduction of the viral load of a coronavirus in a human, wherein the pharmaceutically acceptable salt of 5-amino-2,3-dihydro-1,4-phthalazinedione is 5-amino-2,3-dihydro-1,4-phthalazinedione sodium salt and said coronaviral infection is a SARS-CoV-2 infection.

In another aspect the present application refers to a pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof for use in the prophylaxis or treatment of a coronaviral infection in a human, wherein the pharmaceutically acceptable salt of 5-amino-2,3-dihydro-1,4-phthalazinedione is 5-amino-2,3-dihydro-1,4-phthalazinedione sodium salt and said human is asymptomatic and said coronaviral infection is a SARS-CoV-2 infection.

In another aspect the present application refers to a pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof for use in the prophylaxis or treatment of a coronaviral infection in a human, wherein the pharmaceutically acceptable salt of 5-amino-2,3-dihydro-1,4-phthalazinedione is 5-amino-2,3-dihydro-1,4-phthalazinedione sodium salt and said human shows mild coronavirus-infection related symptoms, is not in need of hospitalization and said coronaviral infection is a SARS-CoV-2 infection.

In another aspect the present application refers to a pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof for use in the treatment of a coronaviral infection in a human, wherein the pharmaceutically acceptable salt of 5-amino-2,3-dihydro-1,4-phthalazinedione is 5-amino-2,3-dihydro-1,4-phthalazinedione sodium salt and said human shows severe coronavirus-infection related symptoms, is in need of hospitalization and said coronaviral infection is a SARS-CoV-2 infection.

In another aspect the present application refers to a pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof for use in the treatment of a coronaviral infection in a human, wherein the pharmaceutically acceptable salt of 5-amino-2,3-dihydro-1,4-phthalazinedione is 5-amino-2,3-dihydro-1,4-phthalazinedione sodium salt and said human shows severe coronavirus-infection related symptoms, is undergoing acute lung injury and said coronaviral infection is a SARS-CoV-2 infection.

More preferred is a pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof for use in the prophylaxis or treatment of a coronaviral infection, wherein said coronaviral infection is SARS-CoV-2.

More preferred is a pharmaceutical combination of 5-amino-2,3-dihydro-1,4-phthalazinedione or one of its pharmaceutically acceptable salts, hydrates or solvates and quinine or a pharmaceutically acceptable salt thereof for use in the prophylaxis or treatment of a coronaviral infection, wherein said coronaviral infection is SARS-CoV-2.

More preferred is a pharmaceutical combination of 5-amino-2,3-dihydro-1,4-phthalazinedione or one of its pharmaceutically acceptable salts, hydrates or solvates and quinidine or pharmaceutically salt thereof for use in the prophylaxis or treatment of a coronaviral infection, wherein said coronaviral infection is SARS-CoV-2.

Most preferred is a pharmaceutical combination of 5-amino-2,3-dihydro-1,4-phthalazinedione sodium salt and a 6'-methoxycinchonan-9-ol or a pharmaceutically acceptable salt thereof for use in the prophylaxis or treatment of a coronaviral infection, wherein said coronaviral infection is SARS-CoV-2.

Most preferred is a pharmaceutical combination of 5-amino-2,3-dihydro-1,4-phthalazinedione sodium salt and quinine or a pharmaceutically acceptable salt thereof for use in the prophylaxis or treatment of a coronaviral infection, wherein said coronaviral infection is SARS-CoV-2.

Most preferred is a pharmaceutical combination of 5-amino-2,3-dihydro-1,4-phthalazinedione sodium salt and quinidine or pharmaceutically salt thereof for use in the prophylaxis or treatment of a coronaviral infection, wherein said coronaviral infection is SARS-CoV-2.

It is understood in the scope of the present application that all beforementioned aspects of the invention are disclosed for a 6'-methoxycinchonan-9-ol or for quinine or for quinidine or a pharmaceutically acceptable salt thereof as combination substance of 5-amino-2,3-dihydro-1,4-phthalazinedione or one of its pharmaceutically acceptable salts, hydrates or solvates, in particular of 5-amino-2,3-dihydro-1,4-phthalazinedione sodium salt.

The other above-mentioned animal coronaviruses have not yet made a transfer to humans (zoonosis), but this may happen in the future with an unpredictable pathology. Thus, the scope of the present application relates also to a pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof for use in the prophylaxis or treatment of these animal coronaviral infections in animals and humans.

The concept for treating coronaviral infections over all species is based on the structural similarity of coronaviruses. Thus, it can be assumed that treatment and/or prevention options can be transferred from one coronavirus to another. Coronavirus particles contain four main structural proteins: spike (S), membrane (M), envelope (E) and nucleocapsid (N), all of which are encoded within the 3' end of the viral genome.

Coronaviruses contain a non-segmented, positive-sense RNA genome of ~30 kb. The genome contains a 5' cap structure along with a 3' poly (A) tail, allowing it to act as an mRNA for translation of the replicase polyproteins. The replicase gene encoding the nonstructural proteins (nsps) occupies two-thirds of the genome, about 20 kb, as opposed to the structural and accessory proteins, which make up only about 10 kb of the viral genome. The organization of the coronavirus genome is 5'-leader-UTR- replicase-S (Spike)-E (Envelope)-M (Membrane)- N (Nucleocapsid)-3' UTR-poly (A) tail with accessory genes interspersed within the structural genes at the 3' end of the genome. The accessory proteins are almost exclusively nonessential for replication in tissue culture; however, some have been shown to have important roles in viral pathogenesis (cf. Zhao et al. (2012) Cell Host Microbe 11: 607-616).

The coronavirus life cycle starts with an initial attachment of the virion to the host cell by interactions between the S protein and its receptor. The sites of receptor binding domains (RBD) within the S1 region of a coronavirus S protein vary depending on the virus. The S-protein-receptor interaction is the primary determinant for a coronavirus to infect a host species and also governs the tissue tropism of the virus. Many coronaviruses utilize peptidases as their cellular receptor. It is unclear why peptidases are used, as entry occurs even in the absence of the enzymatic domain of these proteins. Many alphacoronaviruses utilize aminopeptidase N (APN) as their receptor, many betacoronaviruses such as SARS-CoV, SARS-CoV-2 and HCoV-NL63 use angiotensin-converting enzyme II (ACE2) receptors, MHV enters through CEACAM1, and MERS-CoV binds to dipeptidyl-peptidase 4 (DPP4) to gain entry into human cells. Following receptor binding, the virus must next gain access to the host cell cytosol. This is generally accomplished by acid-dependent proteolytic cleavage of S protein by a cathepsin, TMPRRS2 or another protease, followed by fusion of the viral and cellular membranes, and ultimately the release of the viral genome into the cytoplasm.

Coronaviruses encode either two or three proteases that cleave the replicase polyproteins. They are the papain-like proteases (PLpro), encoded within nsp3, and a serine type protease, the main protease, or Mpro, encoded by nsp5. Most coronaviruses encode two PLpros within nsp3, except the gammacoronaviruses, SARS-CoV and MERS-CoV, which only express one PLpro (Mielech et al. (2014) Virus Res doi: 10.1016).

This papain-like protease (PLpro) was found in SARS-CoV to act the same way as a deubiquitinase within the human cellular ubiquitin proteasome system (UPS) (cf. Raaben et al. (2010) J Virol 84: 7869-7879). PLpro in SARS-CoV-2 has a very high homology with SARS-CoV (96.1%, Nguyen et al. (2020) https://doi:org/10:1101/2020.02.05.936013).

The terms "composition" or "pharmaceutical composition" comprise at least one active ingredient in any pharmacologically acceptable defined dosage and dosage form together with at least one pharmaceutically acceptable excipient, as well as all agents that are generated from the ingredients as outlined below directly or indirectly as a combination, accumulation, complex or crystal, or as a consequence of other reactions or interactions, as well as optionally at least one further pharmaceutical drug, as listed below.

The term "excipient" is used in this application to describe any component of a pharmaceutical composition apart of the pharmaceutically active principle. The selection of suitable excipients depends on a variety of factors, such as the dosage form, the dosage, the desired solubility and the stability of the composition.

The terms "effect", "therapeutic effect", "action", "therapeutic action", "efficacy" and "effectiveness" with regard to the pharmaceutical combination of the disclosure or any other active substance mentioned in the description refers to causally occurring beneficial consequences in the organism to which said substance has been administered before.

According to the invention the terms "effective amount" and "therapeutically effective amount" refer to an amount of the pharmaceutical combination of the disclosure that is sufficiently large to cause a desired beneficial effect in a subject in need of such a treatment.

The terms "treatment" and "therapy" comprise the administration of at least the substance of the invention, alone or in combination with at least one further pharmaceutical drug, independently of the chronological order of the administration. Such an administration is intended to substantially improve the disease course of a coronaviral infection by either completely curing the disease or by stopping or decelerating the increase of disabilities during the course of the disease.

The terms "prophylaxis" or "prophylactic treatment" comprise the administration of at least the substance of the invention, alone or in combination with at least one further pharmaceutical drug, independently of the chronological order of the administration, in order to prevent or suppress the manifestation of symptoms attributed to a coronaviral infection. It refers in particular to medical conditions of a patient in which the manifestation of such symptoms is expected to occur in the far or near future with a reasonable probability.

The terms "subject" and "patient" comprise individuals suffering from disease symptoms or disabilities related to a coronaviral infection wherein said diagnosis is either approved or suspected. Individuals are mammals, in particular humans.

The pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof can be used as a single therapy or can further be combined with at least one further active ingredient selected from a group comprising active ingredients used in disease-modifying therapies for a coronaviral infection, in symptomatic therapies of a coronaviral infection and in the treatment of comorbidities.

The pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof can be used simultaneously, separately or sequentially for treating or preventing disease symptoms. The at least two active agents may be provided in a single dosage form or as separate formulation, each formulation containing at least one of the two active agents. One or any of the active agents may be formulated as a bolus.

In particular, the present application discloses a pharmaceutical combination of 5-amino-2,3-dihydro-1,4-phthalazinedione or one of its pharmaceutically acceptable salts and a 6'-methoxycinchonan-9-ol or a pharmaceutically acceptable salt thereof for use in the treatment of a coronaviral infection, wherein a previous treatment with at least one other pharmaceutically active agent was refractory.

The terms "medicine" or "medical" comprise human as well as veterinary medicine.

The term "organism" refers to a living being, especially a human or an animal, possessing a self-regulating immunological system.

The term "host organism" is used in terms of the application for those organisms exploited for replication by viruses, herein especially retroviruses, following an infection with them.

The term "active agent" in this application refers to 5-amino-2,3-dihydro-1,4-phthalazinedione or one of its pharmaceutically acceptable salts, hydrates or solvates or to quinine or quinidine or pharmaceutically salt thereof, if not stated otherwise. Moreover, this term can comprise further pharmaceutical agents, known from the state of the art.

The terms "composition" and "pharmaceutical composition" comprise a pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof in any pharmacologically suitable defined dose and dosage form together with at least one suitable excipient or carrier substance as well as all substances which are directly or indirectly generated as a combination, accumulation, complex formation or crystal of the aforementioned ingredients, or come into being as a result of other reactions or interactions.

The term "excipient" is used in this application to describe each component of a pharmaceutical composition in addition to the active agent. The selection of a suitable excipient depends on factors such as dosage form and dose as well as the influence on the solubility and stability of the composition by the excipient itself.

The term "action" describes the inherent specific mode of action of the respective agent in the scope of the present application.

The terms "effect", "therapeutic effect", "action", "therapeutic action" regarding at least one active agent according to the invention refer to causally occurring beneficial consequences for the organism, to which the at least one active agent has been administered.

In terms of the application, "therapeutically effective dose" means that a sufficient dose of a pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof is administered to a living being or to a patient in need of such a treatment.

The terms "joint administration", "combined administration" or "simultaneous administration" of at least one pharmaceutical agent according to the invention and/or of at least one pharmaceutical agent from the state of the art comprise the administration of the mentioned agents at the same time or at time points factually related close to each other, as well as administrations of said agents at different times within a coherent experiment. The chronological order of the administration of said agents is not limited by these terms. Those skilled in the art will have no difficulties to deduce the described administrations in respect to their chronological or local order from his knowledge and experience.

The term "living being" refers to every animal, especially vertebrate, including human. A "patient" in terms of the application is a living being who suffers from a definable and diagnosable disease, and to whom a suitable active agent can be administered.

The terms "prophylaxis", "treatment" and "therapy" comprise the administration of a pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof to a living being, in order to prevent the development of a certain disease, to inhibit, and to alleviate the symptoms, or to initiate a healing process of the respective disease.

The pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof can be applied in the prophylaxis or treatment of a coronaviral infection by any medically acceptable administration route to a patient in need thereof. Such medically acceptable administration routes can be e.g., by inhalation, by intubation, orally, parenterally, intraperitoneally, intravenously, intraarterially, intramuscularly, topically, transdermally, subcutaneously, intradermally, sublingually, conjunctivally, intravaginally, rectally or nasally.

Preferred oral formulations for use in the prophylaxis or treatment of a coronaviral infection are capsules or tablets containing 5-amino-2,3-dihydro-1,4-phthalazinedione or one of its pharmaceutically acceptable salts, hydrates and solvates in an amount of 50 mg, 100 mg, 150 mg, 200 mg, 300 mg, 400 mg, 500 mg or 600 mg, preferably 100 mg, 150 mg, 200 mg, 300 mg or 400 mg, most preferably 300 mg and a 6'-methoxycinchonan-9-ol or a pharmaceutically acceptable salt thereof.

Dosages of quinine sulfate are 200 mg, 260 mg, 324 mg or 648 mg (corresponding to 524 mg quinine base) in hard gelatin capsules, preferably 200 mg and 324 mg, most preferably 324 mg, as quinine sulfate in film-coated tablets 200 mg and 300 mg, more preferred 200 mg.

Injectable solutions are available at a dosage of 600 mg quinine dihydrochloride in 2 ml ampoules, corresponding to a concentration of 300 mg/ml.

Dosages of quinidine sulfate are 200 mg and 300 mg in tablets and 300 mg as an extended-release tablet. 200 mg are preferred. Quinidine gluconate extended-release tablets have a strength of 324 g.

Injectable solutions were available with 80 mg/ml quinidine gluconate but were discontinued in the USA.

In the scope of the present application it is understood that any combinations of said dosages of 5-amino-2,3-dihydro-1,4-phthalazinedione or one of its pharmaceutically acceptable salts, hydrates and solvates with quinine or quinidine or one of their pharmaceutically acceptable salts are disclosed.

In another aspect of the invention a pharmaceutical composition for use in the prophylaxis or treatment of a coronaviral infection is disclosed, wherein said pharmaceutical composition contains 5-amino-2,3-dihydro-1,4-phthalazinedione or one of its pharmaceutically acceptable salts, hydrates or solvates and a 6'-methoxycinchonan-9-ol or a pharmaceutically acceptable salt thereof, a carrier and at least one pharmaceutically acceptable excipient.

The term "pharmaceutically acceptable excipient(s)" refers to natural or synthetic compounds that are added to a pharmaceutical formulation alongside the pharmaceutical active agent. They may help to bulk up the formulation, to enhance the desired pharmacokinetic properties or the stability of the formulation, as well as being beneficial in the manufacturing process. Advantageous classes of excipients according to the invention include, carriers, binding agents, colorants, buffers, preservatives, antioxidants, coatings, sweeteners, thickening agents, pH-regulators, acidity regulators, acidifiers, solvents, isotonizing agents, disintegrants, glidants, lubricants, emulsifiers, solubilizing agents, stabilizers, diluents, anti-caking agents (anti-adherents), sorbents, foaming agents, anti-foaming agents, opacifiers, fatliquors, consistency enhancers, hydrotropes, aromatic and flavoring substances.

In general, one or more pharmaceutically acceptable carriers are added to a pharmaceutically active agent. Eligible are all carriers known in the art and combinations thereof. In solid dosage forms they can be for example plant and animal fats, waxes, paraffins, starch, tragacanth, cellulose derivatives, polyethylene glycols, silicones, bentonites, silica, talcum, zinc oxide. For liquid dosage forms and emulsions suitable carriers are for example solvents, solubilizing agents, emulsifiers such as water, ethanol, isopropanol, ethyl carbonate, ethyl acetate, benzyl alcohol, benzyl benzoate, propylene glycol, 1,3-butyl glycol, cotton seed oil, peanut oil, olive oil, castor oil, sesame oil, glycerol fatty acid esters, polyethylglycols, fatty acid esters of sorbitan. Suspensions according to the invention may use carriers known in the art such as diluents (e.g., water, ethanol or propylene glycol), ethoxylated isostearyl alcohols, polyoxyethylene and polyoxyethylene sorbitan esters, microcrystalline cellulose, bentonites, agar agar, tragacanth.

The term binding agents refers to substances that bind powders or glue them together, rendering them cohesive through granule formation. They serve as a "glue" of the formulation. Binding agents increase the cohesive strength of the provided diluent or filler.

Suitable binding agents are for example starch from wheat, corn, rice or potato, gelatin, naturally occurring sugars such as glucose, sucrose or beta-lactose, sweeteners from corn, natural and synthetic gums such as acacia, tragacanth or ammonium calcium alginate, sodium alginate, carboxymethyl cellulose, sodium carboxymethyl cellulose, hydroxypropyl carboxymethyl cellulose, polyethylene glycol, polyvinyl pyrrolidone, magnesium aluminum silicate, waxes and others. The percentage of the binding agent in the composition can range from 1 - 30 % by weight, preferred 2 - 20 % by weight, more preferred 3 - 10 % by weight and most preferred 3 - 6 % by weight.

Colorants are excipients that bestow a colorization to the pharmaceutical formulation. These excipients can be food colorants. They can be adsorbed on a suitable adsorption means such as clay or aluminum oxide. A further advantage of a colorant is that it may visualize spilled aqueous solution on the nebulizer and/or the mouthpiece to facilitate cleaning. The amount of the colorant may vary between 0.01 and 10 % per weight of the pharmaceutical composition, preferred between 0.05 and 6 % per weight, more preferred between 0.1 and 4 % per weight, most preferred between 0.1 and 1 % per weight.

Suitable pharmaceutical colorants are for example curcumin, riboflavin, riboflavin-5'-phosphate, tartrazine, alkannin, quinolione yellow WS, Fast Yellow AB, riboflavin-5'-sodium phosphate, yellow 2G, Sunset yellow FCF, orange GGN, cochineal, carminic acid, citrus red 2, carmoisine, amaranth, Ponceau 4R, Ponceau SX, Ponceau 6R, erythrosine, red 2G, Allura red AC, Indathrene blue RS, Patent blue V, indigo carmine, Brilliant blue FCF, chlorophylls and chlorophyllins, copper complexes of chlorophylls and chlorophyllins, Green S, Fast Green FCF, Plain caramel, Caustic sulfite caramel, ammonia caramel, sulfite ammonia caramel, Black PN, Carbon black, vegetable carbon, Brown FK, Brown HT, alpha-carotene, beta-carotene, gamma-carotene, annatto, bixin, norbixin, paprika oleoresin, capsanthin, capsorubin, lycopene, beta-apo-8'-carotenal, ethyl ester of beta-apo-8'-carotenic acid, flavaxanthin, lutein, cryptoxanthin, rubixanthin, violaxanthin, rhodoxanthin, canthaxanthin, zeaxanthin, citranaxanthin, astaxanthin, betanin, anthocyanins, saffron, calcium carbonate, titanium dioxide, iron oxides, iron hydroxides, aluminum, silver, gold, pigment rubine, tannin, orcein, ferrous gluconate, ferrous lactate.

Moreover, buffer solutions are preferred for liquid formulations, in particular for pharmaceutical liquid formulations. The terms buffer, buffer system and buffer solution, in particular of an aqueous solution, refer to the capacity of the system to resist a pH change by the addition of an acid or a base, or by dilution with a solvent. Preferred buffer systems may be selected from the group comprising formate, lactate, benzoic acid, oxalate, fumarate, aniline, acetate buffer, citrate buffer, glutamate buffer, phosphate buffer, succinate, pyridine, phthalate, histidine, MES (2-(N-morpholino) ethanesulfonic acid), maleic acid, cacodylate (dimethyl arsenate), carbonic acid, ADA (N-(2-acetamido)imino diacetic acid, PIPES (4-piperazine-bis-ethanesulfonic acid), BIS-TRIS propane (1,3-bis[tris(hydroxymethyl)methylamino] propane), ethylene diamine, ACES (2-[(amino-2-oxoethyl)amino]ethanesulfonic acid), imidazole, MOPS (3-(N-morpholino) propanesulfonic acid), diethyl malonic acid, TES (2-[tris(hydroxymethyl)methyl]aminoethanesulfonic acid), HEPES (N-2-hydroxyethylpiperazine-N'-2-ethanesulfonic acid), as well as other buffers with a pKₐ between 3.8 and 7.7.

Preferred are carbonic acid buffers such as acetate buffer and dicarboxylic acid buffers such as fumarate, tartrate and phthalate as well as tricarboxylic acid buffers such as citrate.

A further group of preferred buffers are inorganic buffers such as sulfate hydroxide, borate hydroxide, carbonate hydroxide, oxalate hydroxide, calcium hydroxide and phosphate buffers. Another group of preferred buffers are nitrogen-containing puffers such as imidazole, diethylene diamine and piperazine. Furthermore preferred are sulfonic acid buffers such as TES, HEPES, ACES, PIPES, [(2-hydroxy-1,1-bis-(hydroxymethyl)ethyl)amino]-1-propanesulfonic acid (TAPS), 4-(2-hydroxyethyl)piperazine-1-propanesulfonic acid (EEPS), MOPS and *N*,*N*-bis-(2-hydroxyethyl)-2-aminoethanesulfonic acid (BES). Another group of preferred buffers are glycine, glycyl-glycine, glycyl-glycyl-glycine, N,N-bis-(2-hydroxyethyl)glycine and *N*-[2-hydroxy-1,1-bis(hydroxymethyl)ethyl]glycine (tricine). Preferred are also amino acid buffers such as glycine, alanine, valine, leucine, isoleucine, serine, threonine, phenylalanine, tyrosine, tryptophan, lysine, arginine, histidine, aspartate, glutamate, asparagine, glutamine, cysteine, methionine, proline, 4-hydroxy proline, *N,N,N-*trimethyllysine, 3-methyl histidine, 5-hydroxy lysine, o-phosphoserine, gamma-carboxyglutamate, [epsilon]-*N*-acetyl lysine, [omega]-*N*-methyl arginine, citrulline, ornithine and their derivatives. Particularly preferred is KH₂PO₄ buffer.

Preservatives for liquid and/or solid dosage forms can be used on demand. They may be selected from the group comprising, but not limited to, sorbic acid, potassium sorbate, sodium sorbate, calcium sorbate, methyl paraben, ethyl paraben, methyl ethyl paraben, propyl paraben, benzoic acid, sodium benzoate, potassium benzoate, calcium benzoate, heptyl p-hydroxybenzoate, sodium methyl para-hydroxybenzoate, sodium ethyl para-hydroxybenzoate, sodium propyl para-hydroxybenzoate, benzyl alcohol, benzalkonium chloride, phenylethyl alcohols, cresols, cetylpyridinium chloride, chlorobutanol, thiomersal (sodium 2-(ethylmercurithio) benzoic acid), sulfur dioxide, sodium sulfite, sodium bisulfite, sodium metabisulfite, potassium metabisulfite, potassium sulfite, calcium sulfite, calcium hydrogen sulfite, potassium hydrogen sulfite, biphenyl, orthophenyl phenol, sodium orthophenyl phenol, thiabendazole, nisin, natamycin, formic acid, sodium formate, calcium formate, hexamine, formaldehyde, dimethyl dicarbonate, potassium nitrite, sodium nitrite, sodium nitrate, potassium nitrate, acetic acid, potassium acetate, sodium acetate, sodium diacetate, calcium acetate, ammonium acetate, dehydroacetic acid, sodium dehydroacetate, lactic acid, propionic acid, sodium propionate, calcium propionate, potassium propionate, boric acid, sodium tetraborate, carbon dioxide, malic acid, fumaric acid, lysozyme, copper-(II)-sulfate, chlorine, chlorine dioxide and other suitable substances or compositions known to the person skilled in the art.

The addition of a sufficient amount of antioxidants is particularly preferable for liquid and topical dosage forms. Suitable examples for antioxidants include sodium metabisulfite, alpha-tocopherol, ascorbic acid, maleic acid, sodium ascorbate, ascorbyl palmitate, butylated hydroxyanisole, butylated hydroxytoluene, fumaric acid or propyl gallate. Preferred is the use of sodium metabisulfite, alpha-tocopherol and ascorbyl palmitate.

Tablets or pills are usually coated i.e., the coating constitutes the outer layer. This can be a film coating, a sugar coating with saccharides and a compression coating. Pharmaceutically acceptable varnishes or waxes, HPMC (hydroxypropylmethylcellulose), MC (methylcellulose) or HPC (hydroxypropylcellulose) can be used. Such a coating may help to disguise the taste, to ease the swallowing or the identification. Often plasticizers and pigments are included in the coating. Capsules normally have a gelatinous envelope that encloses the pharmaceutical composition according to the disclosure. The specific composition and thickness of this gelatinous layer determines how fast absorption takes place after ingestion of the capsule. Of special interest are sustained release formulations, as known in the art.

Suitable sweeteners can be selected from the group comprising mannitol, glycerol, acesulfame potassium, aspartame, cyclamate, isomalt, isomaltitol, saccharin and its sodium, potassium and calcium salts, sucralose, alitame, thaumatin, glycyrrhizin, neohesperidine dihydrochalcone, steviol glycosides, neotame, aspartame-acesulfame salt, maltitol, maltitol syrup, lactitol, xylitol, erythritol.

Suitable thickening agents can be selected from the group comprising, but not limited to, polyvinyl pyrrolidone, methyl cellulose, hydroxypropyl methyl cellulose, hydroxypropyl cellulose, dextrins, polydextrose, modified starch, alkaline modified starch, bleached starch, oxidized starch, enzyme-treated starch, monostarch phosphate, distarch phosphate esterified with sodium trimetaphosphate or phosphorus oxychloride, phosphate distarch phosphate, acetylated distarch phosphate, starch acetate esterified with acetic anhydride, starch acetate esterified with vinyl acetate, acetylated distarch adipate, acetylated distarch glycerol, distarch glycerin, hydroxypropyl starch, hydroxy propyl distarch glycerin, hydroxypropyl distarch phosphate, hydroxypropyl distarch glycerol, starch sodium octenyl succinate, acetylated oxidized starch, hydroxyethyl cellulose.

Suitable pH-regulators for liquid dosage forms are e.g., sodium hydroxide, hydrochloric acid, buffer substances such as sodium dihydrogen phosphate or disodium hydrogenphosphate.

Suitable acidity regulators can be selected from the group comprising acetic acid, potassium acetate, sodium acetate, sodium diacetate, calcium acetate, carbon dioxide, malic acid, fumaric acid, sodium lactate, potassium lactate, calcium lactate, ammonium lactate, magnesium lactate, citric acid, mono-, di-, trisodium citrate, mono-, di-, tripotassium citrate, mono-, di-, tricalcium citrate, tartaric acid, mono-, disodium tartrate, mono-, dipotassium tartrate, sodium potassium tartrate, ortho-phosphoric acid, lecithin citrate, magnesium citrate, ammonium malate, sodium malate, sodium hydrogen malate, calcium malate, calcium hydrogen malate, adipic acid, sodium adipate, potassium adipate, ammonium adipate, succinic acid, sodium fumarate, potassium fumarate, calcium fumarate, ammonium fumarate, 1,4-heptonolactone, triammonium citrate, ammonium ferric citrate, calcium glycerophosphate, isopropyl citrate, potassium carbonate, potassium bicarbonate, ammonium carbonate, ammonium bicarbonate, magnesium carbonate, magnesium bicarbonate, ferrous carbonate, ammonium sulfate, aluminum potassium sulfate, aluminum ammonium sulfate, sodium hydroxide, potassium hydroxide, ammonium hydroxide, magnesium hydroxide, gluconic acid.

Acidifiers use to be inorganic chemicals that either produce or become acid. Suitable examples are: Ammonium chloride, calcium chloride.

Suitable solvents may be selected from the group comprising, but not limited to, water, carbonated water, water for injection, water with isotonizing agents, saline, isotonic saline, alcohols, particularly ethyl and n-butyl alcohol, and mixtures thereof.

Suitable isotonizing agents are for example pharmaceutically acceptable salts, in particular sodium chloride and potassium chloride, sugars such as glucose or lactose, sugar alcohols such as mannitol and sorbitol, citrate, phosphate, borate and mixtures thereof.

Suitable disintegrants can be selected from the group comprising starch, cold water-soluble starches such as carboxymethyl starch, cellulose derivatives such as methyl cellulose and sodium carboxymethyl cellulose, microcrystalline cellulose and cross-linked microcrystalline celluloses such as croscarmellose sodium, natural and synthetic gums such as guar, agar, karaya (Indian tragacanth), locust bean gum, tragacanth, clays such as bentonite, xanthan gum, alginates such as alginic acid and sodium alginate, foaming compositions i.a. Moisture expansion is supported by for example starch, cellulose derivatives, alginates, polysaccharides, dextrans, cross-linked polyvinyl pyrrolidone. The amount of the disintegrant in the composition may vary between 1 and 40% per weight, preferred between 3 and 20% per weight, most preferred between 5 and 10% per weight.

Glidants are materials that prevent a baking of the respective supplements and improve the flow characteristics of granulations so that the flow is smooth and constant. Suitable glidants comprise silicon dioxide, magnesium stearate, sodium stearate, starch and talcum. The amount of the glidant in the composition may vary between 0.01 and 10% per weight, preferred between 0.1 and 7% per weight, more preferred between 0.2 and 5% per weight, most preferred between 0.5 and 2% per weight.

The term "lubricants" refers to substances that are added to the dosage form for facilitating tablets, granulates etc. to be released from the press mold or the outlet nozzle. They diminish friction or abrasion. Lubricants are usually added shortly before pressing, as they should be present on the surface of the granules and between them and the parts of the press mold. The amount of the lubricant in the composition may vary between 0.05 and 15% per weight, preferred between 0.2 and 5% per weight, more preferred between 0.3 and 3% per weight, most preferred between 0.3 and 1.5% per weight. Suitable lubricants are i.a. sodium oleate, metal stearates such as sodium stearate, calcium stearate, potassium stearate and magnesium stearate, stearic acid, sodium benzoate, sodium acetate, sodium chloride, boric acid, waxes having a high melting point, polyethylene glycol.

Emulsifiers can be selected for example from the following anionic and non-ionic emulsifiers: Anionic emulsifier waxes, cetyl alcohol, cetylstearyl alcohol, stearic acid, oleic acid, polyoxyethylene polyoxypropylene block polymers, addition products of 2 to 60 mol ethylene oxide to castor oil and/or hardened castor oil, wool wax oil (lanolin), sorbitan esters, polyoxyethylene alkyl esters, polyoxyethylene sorbitan fatty acid esters, polyoxyethene sorbitan monolaurate, polyoxyethene sorbitan monooleate, polyoxyethene sorbitan monopalmitate, polyoxyethene sorbitan monostearate, polyoxyethene sorbitan tristearate, polyoxyethene stearate, polyvinyl alcohol, metatartaric acid, calcium tartrate, alginic acid, sodium alginate, potassium alginate, ammonium alginate, calcium alginate, propane-1,2-diol alginate, carrageenan, processed Eucheuma seaweed, locust bean gum, tragacanth, acacia gum, karaya gum, gellan gum, gum ghatti, glucomannane, pectin, amidated pectin, ammonium phosphatides, brominated vegetable oil, sucrose acetate isobutyrate, glycerol esters of wood rosins, disodium phosphate, trisodium diphosphate, tetrasodium diphosphate, dicalcium diphosphate, calcium dihydrogen diphosphate, sodium triphosphate, pentapotassium triphosphate, sodium polyphosphates, sodium calcium polyphosphate, calcium polyphosphates, ammonium polyphosphate, beta-cyclodextrin, powdered cellulose, methyl cellulose, ethyl cellulose, hydroxypropyl cellulose, hydroxypropyl methylcellulose, ethyl methyl cellulose, carboxymethyl cellulose, sodium carboxymethyl cellulose, ethyl hydroxyethyl cellulose, croscarmellose, enzymically hydrolyzed carboxymethyl cellulose, mono- and diglycerides of fatty acids, glyceryl monostearate, glyceryl distearate, acetic acid esters of mono- and diglycerides of fatty acids, lactic acid esters of mono- and diglycerides of fatty acids, citric acid esters of mono- and diglycerides of fatty acids, tartaric acid esters of mono- and diglycerides of fatty acids, mono- and diacetyl tartaric acid esters of mono- and diglycerides of fatty acids, mixed acetic and tartaric acid esters of mono- and diglycerides of fatty acids, succinylated monoglycerides, sucrose esters of fatty acids, sucroglycerides, polyglycerol esters of fatty acids, polyglycerol polyricinoleate, propane-1,2-diol esters of fatty acids, propylene glycol esters of fatty acids, lactylated fatty acid esters of glycerol and propane-1, thermally oxidized soy bean oil interacted with mono- and diglycerides of fatty acids, dioctyl sodium sulphosuccinate, sodium stearoyl-2-lactylate, calcium stearoyl-2-lactylate, stearyl tartrate, stearyl citrate, sodium stearoyl fumarate, calcium stearoyl fumarate, stearyl tartrate, stearyl citrate, sodium stearoyl fumarate, calcium stearoyl fumarate, sodium laurylsulfate, ethoxylated mono- and diglycerides, methyl glucoside-coconut oil ester, sorbitan monostearate, sorbitan tristrearate, sorbitan monolaurate, sorbitan monooleate, sorbitan monopalmitate, sorbitan trioleate, calcium sodium polyphosphate, calcium polyphosphate, ammonium polyphosphate, cholic acid, choline salts, distarch glycerol, starch sodium octenyl succinate, acetylated oxidized starch. Preferred are glycerin monooleate, stearic acid, phospholipids such as lecithin.

Suitable as surface-active solubilizing agents (solubilizers) are for example diethylene glycol monoethyl ester, polyethyl propylene glycol co-polymers, cyclodextrins such as α- and β-cyclodextrin, glyceryl monostearates such as Solutol HS 15 (Macrogol-15-hydroxystearate from BASF, PEG 660-15 hydroxystearates), sorbitan esters, polyoxyethylene glycol, polyoxyethylene sorbitanic acid esters, polyoxyethylene sorbitan monooleate, polyoxyethylene oxystearic acid triglyceride, polyvinyl alcohol, sodium dodecyl sulfate, (anionic) glyceryl monooleates.

Stabilizers are substances that can be added to prevent unwanted changes. Though stabilizers are not real emulsifiers they may also contribute to the stability of emulsions. Suitable examples for stabilizers are oxystearin, xanthan gum, agar, oat gum, guar gum, tara gum, polyoxyethene stearate, aspartame-acesulfame salt, amylase, proteases, papain, bromelain, ficin, invertase, polydextrose, polyvinyl pyrrolidone, polyvinyl polypyrrolidone, triethyl citrate, maltitol, maltitol syrup.

Diluents or fillers are inactive substances added to drugs for handling minimal amounts of active agents. Examples for suitable diluents are water, mannitol, pre-gelatinized starch, starch, microcrystalline cellulose, powdered cellulose, silicified microcrystalline cellulose, dibasic calcium phosphate dihydrate, calcium phosphate, calcium carbonate, hydroxypropyl cellulose, hydroxyethyl cellulose, hydroxypropyl methylcellulose, polyethylene glycol, xanthan gum, gum arabic or any combination thereof.

Anti-caking agents (anti-adherents) can be added to a supplement or a composition of supplements for preventing the formation of lumps and for easing packaging, transport, release from the at least one chamber of the dispensing cap and consumption. Suitable examples include tricalcium phosphate, powdered cellulose, magnesium stearate, sodium bicarbonate, sodium ferrocyanide, potassium ferrocyanide, calcium ferrocyanide, bone phosphate, sodium silicate, silicon dioxide, calcium silicate, magnesium trisilicate, talcum powder, sodium aluminosilicate, potassium aluminum silicate, calcium aluminosilicate, bentonite, aluminum silicate, stearic acid, polydimethyl siloxane.

Sorbents are materials that soak up oil from the water. Suitable examples include natural sorbents such as peat moss, sawdust, feathers, and anything else natural that contains carbon and synthetic sorbents such as polyethylene and nylon. Sorbents are used for tablet/capsule moisture-proofing by limited fluid sorbing (taking up of a liquid or a gas either by adsorption or by adsorption) in a dry state.

In some galenic formulations it may be desirable that a liquid oral dosage form generates some foam on being dissolved. Such an effect can be supported through the addition of a foaming agent that reduces the surface tension of the liquid, thus facilitating the formation of bubbles, or it increases its colloidal stability by inhibiting coalescence of bubbles. Alternatively, it may stabilize foam. Suitable examples include mineral oil, quillaia extract, triethyl citrate, sodium lauryl ether sulfate, sodium lauryl sulfate, ammonium lauryl sulfate.

Alternatively, some liquid oral dosage forms may appear slightly foamy upon preparation. Though this does not interfere with the desired application it may affect patient compliance in case of a medication or the commercial success in case of dietary supplements. Therefore, it may be desirable to add a pharmaceutically acceptable anti-foaming agent (defoamer).

Examples are polydimethylsiloxane or silicone oil in dietary supplements or simethicone in pharmaceuticals.

Opacifiers are substances that render the liquid dosage for, opaque, if desired. They must have a refractive index substantially different from the solvent, in most cases here water. At the same time, they should be inert to the other components of the composition. Suitable examples include titanium dioxide, talc, calcium carbonate, behenic acid, cetyl alcohol, or mixtures thereof.

Suitable fatliquors are e.g., oleic acid decyl ester, hydrated castor oil, light mineral oil, mineral oil, polyethylene glycol, sodium laurylsulfate.

Consistency enhancers are e.g., cetyl alcohol, cetyl ester wax, hydrated castor oil, microcrystalline waxes, non-ionic emulsifier waxes, beeswax, paraffin or stearyl alcohol.

Suitable hydrotropes are alcohols such as ethanol, isopropyl alcohol or polyols such as glycerin.

Suitable aromatic and flavoring substances comprise above all essential oils that can be used for this purpose. In general, this term refers to volatile extracts from plants or parts of plants with the respective characteristic smell. They can be extracted from plants or parts of plants by steam distillation.

Suitable examples are: Essential oils, respectively aromatic substances from achillea, sage, cedar, clove, chamomile, anise, aniseed, star anise, thyme, tea tree, peppermint, mint oil, menthol, cineol, borneol, zingerol, eucalyptus, mango, figs, lavender oil, chamomile blossoms, pine needle, cypress, orange, rose, rosewood, plum, currant, cherry, birch leaves, cinnamon, lime, grapefruit, tangerine, juniper, valerian, lemon, lemon balm, lemon grass, palmarosa, cranberry, pomegranate, rosemary, ginger, pineapple, guava, echinacea, ivy leave extract, blueberry, kaki, melon, alpha- or beta-pinene, alpha-pinene oxide, alpha-campholenic aldehyde, alpha-citronellol, alpha-isoamyl-cinnamic, alpha-cinnamic terpinene, alpha-terpineol, alpha-terpinene, aldehyde C₁₆, alpha-phellandrene, amyl cinnamic aldehyde, amyl salicylate, anisic aldehyde, basil, anethole, bay, benzyl acetate, benzyl alcohol, bergamot, bitter orange peel, black pepper, calamus, camphor, cananga oil, cardamom, carnation, carvacrol, carveol, cassia, castor, cedarwood, cinnamaldehyde, cinnamic alcohol, cis-pinane, citral, citronella, citronellal, citronellol dextro, citronellol, citronellyl acetate; citronellyl nitrile, citrus unshiu, clary sage, clove bud, coriander, corn, cotton seed, d-dihydrocarvone, decyl aldehyde, diethyl phthalate, dihydroanethole, dihydrocarveol, dihydrolinalool, dihydromyrcene, dihydromyrcenol, dihydromyrcenyl acetate; dihydroterpineol, dimethyl salicylate, dimethyloctanal, dimethyloctanol, dimethyloctanyl acetate, diphenyl oxide, dipropylene glycol, d-limonene, d-pulegone, estragole, ethyl vanillin, eucalyptol; eucalyptus citriodora, eucalyptus globulus, eugenol, evening primrose, fenchol, fennel, ferniol, fish, florazon, galaxolide, geraniol, geranium, geranyl acetate, geranyl nitrile, guaiacol, guaiacwood, gurjun balsam, heliotropin, herbanate, hiba, hydroxycitronellal, i-carvone, i-methyl acetate, ionone, isobutyl quinoleine, isobornyl acetate, isobornyl methylether, isoeugenol, isolongifolene, jasmine, lavender, limonene, linallol oxide, linallol, linalool, linalyl acetate, linseed, litsea cubeba, I-methyl acetate, longifolene, mandarin, mentha, menthane hydroperoxide, menthol crystals, menthol laevo, menthone laevo, methyl anthranilate, methyl cedryl ketone, methyl chavicol, methyl hexyl ether, methyl ionone, methyl salicylate, mineral, mint, musk ambrette, musk ketone, musk xylol, myrcene, nerol, neryl acetate, nonyl aldehyde, nutmeg, orris root, para-cymene, parahydroxy phenyl butanone crystals, patchouli, p-cymene, pennyroyal oil, pepper, perillaldehyde, petitgrain, phenyl ethyl alcohol, phenyl ethyl propionate, phenyl ethyl-2methylbutyrate, pimento berry, pimento leaf, pinane hydroperoxide, pinanol, pine ester, pine, pinene, piperonal, piperonyl acetate, piperonyl alcohol, plinol, plinyl acetate, pseudo ionone, rhodinol, rhodinyl acetate, rosalin, ryu, sandalwood, sandenol, sassafras, sesame, soybean, spearmint, spice, spike lavender, spirantol, starflower, tea seed, terpenoid, terpineol, terpinolene, terpinyl acetate, tert-butylcyclohexyl acetate, tetrahydrolinalool, tetrahydrolinalyl acetate, tetrahydromyrcenol, thulasi, thymol, tomato, trans-2-hexenol, trans-anethole, turmeric, turpentine, vanillin, vetiver, vitalizair, white cedar, white grapefruit, wintergreen etc. or mixtures thereof, as well as mixtures of menthol, peppermint and star anise oil or menthol and cherry flavor.

These aromatic or flavoring substances can be included in the range of 0.0001 to 10 % per weight (particularly in a composition), preferred 0.001 to 6% per weight, more preferred 0.001 to 4% per weight, most preferred 0.01 to 1% per weight, with regard to the total composition. Application- or single case-related it may be advantageous to use differing quantities.

According to the invention all the beforementioned excipients and classes of excipients can be used without limitation alone or in any conceivable combination thereof, as long as the inventive use is not thwarted, toxic actions may occur, or respective national legislations are infracted.

In another aspect of the invention the present application relates to a pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof or a pharmaceutical composition according to the invention for use in a formulation for oral administration in the prophylaxis or treatment of a coronaviral infection.

Pharmaceutical formulations suitable for oral dosage forms of a pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof may be administered as separate units such as tablets, soft gelatin capsules, hard gelatin capsules, sugar-coated tablets or pills; powders or granulates; juices, syrups, drops, teas, solutions or suspensions in aqueous or non-aqueous liquids; edible foams or mousses; or in oil-in-water or water-in-oil in emulsions.

In oral dosage forms such as tablets or capsules the active agent can thus be combined with a non-toxic and pharmaceutically acceptable inert carrier such as ethanol, glycerol or water. Powders are produced by grinding the compound to a suitably tiny particle size and mixing them with a pharmaceutical carrier in a similar manner e.g., an edible carbohydrate such as starch or mannitol. A flavor, preservative, dispersant or colorant can also be present.

Tablets are formulated by producing, granulating or dry-pressing a powder mixture, adding a lubricant and a disintegrants and pressing the mixture to a tablet. A powder mixture is produced by mixing a suitably ground compound with a diluent or a base as described before, and if applicable, with a binding agent such as carboxymethyl cellulose, an alginate, gelatin or polyvinyl pyrrolidone, a dissolution retardant, such as, for example, paraffin, an absorption accelerator, such as, for example, a quaternary salt, and/or an absorbent, such as, for example, bentonite, kaolin or dicalcium phosphate. The powder mixture can be granulated by wetting it with a binder, such as, for example, syrup, starch paste, acacia mucilage or solutions of cellulose or polymer materials and pressing it through a sieve. As an alternative to granulation, the powder mixture can be run through a tableting machine, giving lumps of non-uniform shape which are broken up to form granules. The granules can be lubricated by addition of stearic acid, a stearate salt, talc or mineral oil in order to prevent sticking to the tablet casting mold. The lubricated mixture is then pressed to give tablets. The compounds according to the invention can also be combined with a free-flowing inert excipient and then pressed directly to give tablets without carrying out the granulation or dry-pressing steps.

In another aspect of the invention a pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof is provided in hard gelatin capsules. They are fabricated by producing a powder mixture as described before and filling it into shaped gelatin covers. Glidants and lubricants such as highly dispersed silica, talcum, magnesium stearate, calcium stearate or polyethylene glycol can be added to the powder mixture as solids. A disintegrant or solubilizer such as agar agar, calcium carbonate or sodium carbonate can be added likewise for improve the availability of the medication after intake of the capsule. Additionally, suitable binding agents and/or colorants can be added to the mixture, if desirable or necessary.

In another aspect of the invention a pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof is included in soft gelatin capsules (SGC). SGC are dissolved on their passage through the gastrointestinal tract. They consist mainly of gelatin enriched with variable amounts of plasticizers such as glycerol or sorbitan. The release rate depends on the specific formulation of the SGC carrier material. They are also suitable for a sustained release of the active agent. SGC are particularly useful for the administration of poorly water-soluble active agents.

In another aspect of the invention a pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof is included in chewable tablets or hard caramels. Herein the substance is integrated into the matrix of the tablets or caramels.

In another aspect of the invention the present application relates to a pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof or a pharmaceutical composition according to the invention for use in the prophylaxis or treatment of a coronaviral infection in a formulation for inhalatory administration.

For an effective prophylactic or therapeutic treatment of a coronaviral infection that may cause pneumonia, pulmonary edema and/or acute lung injury it is advantageous that the pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof, respectively the pharmaceutical composition according to the invention reaches the patient's alveoli. Therefore, the particle size must be sufficiently small to reach the lowest parts of the airways of the pulmonary tissue. The best inhalatory device class for inhalatory application of a pharmaceutically active agent are the so-called mesh nebulizers. In the scope of the present application practically all mesh nebulizers known in the art can be used, from rather simple single-use mesh nebulizers for cough and cold or for fancy purposes to sophisticated high-end mesh nebulizers for clinical or domestic treatment of serious diseases or conditions of the lower airways.

Suitable commercially available mesh nebulizers, jet nebulizers, ultrasonic nebulizers, dry powder inhalers and (pressurized) metered-dose inhalers comprise, without being limiting, PARI eFlow^{®}rapid, PARI LC STAR^{®}, PARI Velox and PARI Velox Junior (PARI GmbH, Starnberg, Germany), Philips Respironics I-neb and Philips InnoSpire Go (Koninklijke Philips N.V., Eindhoven, Netherlands), VENTA-NEB^{®}-ir, OPTI-NEB^{®}, M-neb^{®} dose⁺ mesh nebulizer inhalation MN-300/8, M-Neb Flow+ and M-neb^{®} mesh nebulizer MN-300/X (NEBU-TEC, Eisenfeld, Germany), Hcmed Deepro HCM-86C and HCM860 (HCmed Innovations Co., Ltd, Taipei, Taiwan), OMRON MicroAir U22 and U100 (OMRON, Kyoto, Japan), Aerogen^{®} Solo, Aerogen^{®} Ultra and Aerogen^{®} PRO (Aerogen, Galway, Ireland), KTMED NePlus NE-SM1 (KTMED Inc., Seoul, South Korea), Vectura Bayer Breelib^{™} (Bayer AG, Leverkusen, Germany), Vectura Fox, MPV Truma and MicroDrop^{®} Smarty (MPV MEDICAL GmbH, Kirchheim, Germany), MOBI MESH (APEX Medical, New Taipei City, Taiwan), B.Well WN-114, TH-134 and TH-135 (B.Well Swiss AG, Widnau, Switzerland), Babybelle Asia BBU01 (Babybelle Asia Ltd., Hongkong), CA-MI Kiwi and others (CA-MI sri, Langhirano, Italy), Diagnosis PRO MESH (Diagnosis S.A., Biatystok, Poland), DIGI O₂ (DigiO₂ International Co., Ltd., New Taipei City, Taiwan), feellife AIR PLUS, AEROCENTRE+, AIR 360+, AIR GARDEN, AIRICU, AIR MASK, AIRGEL BOY, AIR ANGEL, AIRGEL GIRL and AIR PRO 4 (Feellife Health Inc., Shenzhen, China), Hannox MA-02 (Hannox International Corp., Taipei, Taiwan), Health and Life HL100 and HL100A (HEALTH & LIFE Co., Ltd., New Taipei City, Taiwan), Honsun NB-810B (Honsun Co., Ltd., Nantong, China), K-jump^{®} KN-9100 (K-jump Health Co., Ltd., New Taipei City, Taiwan), microlife NEB-800 (Microlife AG, Widnau, Switzerland), OK Biotech Docspray (OK Biotech Co., Ltd., Hsinchu City, Taiwan), Prodigy Mini-Mist^{®} (Prodigy Diabetes Care, LLC, Charlotte, USA), Quatek NM211, NE203, NE320 and NE403 (Big Eagle Holding Ltd., Taipei, Taiwan), Simzo NBM-1 and NBM-2 (Simzo Electronic Technology Ltd., Dongguan, China), Mexus^{®} BBU01 and BBU02 (Tai Yu International Manufactory Ltd., Dongguan, China), TaiDoc TD-7001 (TaiDoc Technology Co., New Taipei City, Taiwan), Vibralung^{®} and HIFLO Miniheart Circulaire II (Westmed Medical Group, Purchase, USA), KEJIAN (Xuzhou Kejian Hi-Tech Co., Ltd., Xuzhou, China), YM-252, P&S-T45 and P&S-360 (TEKCELEO, Valbonne, France), Maxwell YS-31 (Maxwell India, Jaipur, India), Kernmed^{®} JLN-MB001 (Kernmed, Durmersheim, Germany).

Preferred are mesh nebulizers with a piezoelectric activation of the nebulization process, respectively vibrating mesh nebulizers.

Thus, in another aspect of the invention the present application relates to a pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof or a pharmaceutical composition according to the invention for use in the prophylaxis or treatment of a coronaviral infection in a formulation for inhalatory administration, wherein the inhalatory administration is carried out by means of a vibrating mesh nebulizer.

Mesh nebulizers can be classified into two groups according to patient interaction: Continuous mode devices and trigger-activated devices. In continuous mode mesh nebulizers, the nebulized aerosol is continuously released into the mouthpiece and the patient inhales the provided aerosol. In trigger-activated devices a defined amount of aerosol is released only upon an active and deep inspiratory breath. This way a far larger amount of active agent-containing aerosol is inhaled and reaches the lowest airways than with continuous mode devices. The latter lose a large amount of active agent-containing aerosol either to the surrounding or on the passage of the upper airways, as the aerosol release is not coupled to the respiratory cycle.

Therefore, trigger-activated mesh nebulizers are preferred, particularly vibrating mesh nebulizers.

Particularly preferred are trigger-activated mesh nebulizers with a piezoelectric activation of the nebulization process.

Preferred are the mesh nebulizer models PARI eFlow^{®}rapid, Philips Respironics I-neb, Philips InnoSpire Go, M-neb^{®} dose⁺ mesh nebulizer inhalation MN-300/8, Hcmed Deepro HCM-86C and HCM860, OMRON MicroAir U100, Aerogen^{®} Solo, KTMED NePlus NE-SM1, Vectura Fox, Vectura Bayer Breelib^{™}.

The most preferred vibrating mesh nebulizer models are high-end models such as PARI eFlow^{®}rapid, PARI Velox, Philips Respironics I-neb, M-neb^{®} dose⁺ mesh nebulizer inhalation MN-300/8, Aerogen^{®} Solo, Vectura Fox, Vectura Bayer Breelib^{™}.

The mean droplet size is usually characterized as MMAD (median mass aerodynamic diameter). The individual droplet size is referred to as MAD (mass aerodynamic diameter). This value indicates the diameter of the nebulized particles (droplets) at which 50% are smaller or larger, respectively. Particles with a MMAD > 10 µm normally do not reach the lower airways, they often get stuck in the throat. Particles with a MMAD > 5 µm and < 10 µm usually reach the bronchi but not the alveoli. Particles between 100 nm and 1 µm MMAD do not deposit in the alveoli and are exhaled immediately. Therefore, the optimal range is between 1 µm and 5 µm MMAD. Recent publications even favor a narrower range between 3.0 µm and 4.0 µm (cf. Amirav et al. (2010) J Allergy Clin Immunol 25: 1206-1211; Haidl et al. (2012) Pneumologie 66: 356-360).

A further commonly accepted quality parameter is the percentage of the particles in the generated aerosol with a diameter in the range of 1 µm to 5 µm (FPM; fine particle mass). FPM is a measure for the particle distribution. It is calculated by subtracting the percentage of the particles in the generated aerosol with a diameter in the range < 1 µm from the overall percentage of the particles in the generated aerosol with a diameter in the range < 5 µm (FPF; fine particle fraction).

In another aspect of the invention the present application refers also to a method for producing an aerosol according to the invention for the prophylaxis or treatment of a coronaviral infection, comprising the following steps:
a) filling 0.1 ml to 5 ml of an aqueous solution containing a pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof, respectively a pharmaceutical composition according to the invention and optionally at least one pharmaceutically acceptable excipient into the nebulization chamber of a mesh nebulizer,
b) starting vibration of the mesh of the mesh nebulizer at a frequency of 80 kHz to 200 kHz, and
c) discharging the generated aerosol at the side of the mesh of the mesh nebulizer opposite to the nebulization chamber.

The vibration frequency of vibrating mesh nebulizers is normally in the range of 80 kHz to 200 kHz, preferred 90 kHz to 180 kHz, more preferred 100 kHz to 160 kHz, most preferred 105 kHz to 130 kHz (cf. Chen, *The Aerosol Society.* **DDL2019;** Gardenshire et al. (2017) A Guide to Aerosol Delivery Devices for Respiratory Therapists, 4th ed.).

Thus, the beforementioned method is also disclosed with said vibration frequency ranges.

The method according to the invention is thus characterized in that at least 80 % in weight, preferred at least 85 % in weight, most preferred at least 90 % in weight of the pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof or the pharmaceutical composition according to the invention contained in said aqueous solution are nebulized in the generated aerosol.

The method of the invention is particularly effective in nebulizing a high percentage of the pharmaceutically active agent(s) from the provided aqueous solution during a short time. This is an important feature for patient compliance. A considerable percentage of the patient population finds the inhalatory process to be uncomfortable, weary and physically demanding. On the other hand, the patient's active cooperation is essential for an effective and targeted inhalatory application. Therefore, it is desirable that a therapeutically sufficient amount is applied during a period of time as short as possible. Surprisingly, it showed that during a three minutes' time span 95 % of the substance provided in the aqueous solution could be nebulized. This is an ideal time span for a high patient compliance.

Therefore, the method according to the invention is thus characterized in that at least 80 % of the generated aerosol are produced during three minutes after starting nebulization in the mesh nebulizer, preferred at least 85 % and most preferred at least 90 %.

While the pharmaceutically active agent is usually provided in a single dosage container for every nebulization procedure the nebulizer and/or the mouthpiece can be used over a certain time span and need to be replaced at certain intervals. A cleaning of the nebulizer and the mouthpiece is recommended by default after each nebulization. But herein patient compliance cannot be reasonably taken for granted. But even after a meticulous cleaning there are always some deposits of the aerosol in the nebulization chamber, the outlet and/or the mouthpiece. As the aerosol is produced from an aqueous solution these depositions bear the risk of producing a bioburden of bacteria that might contaminate the inhaled aerosol. Deposits may also plug holes in the mesh membrane of the mesh nebulizer. In general, the nebulizer and/or the mouthpiece should be exchanged every one or two weeks. Therefore, it is convenient to offer the medication and the nebulizer as a combined product.

Thus, in another aspect of the invention the present application refers also to a kit comprising a mesh nebulizer and a pharmaceutically acceptable container with an aqueous solution containing an effective amount of a pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof or a pharmaceutical composition according to the invention for the prophylaxis or treatment of a coronaviral infection and optionally at least one pharmaceutically acceptable excipient.

In an alternative kit a pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof or a pharmaceutical composition according to the invention is not provided in form of an aqueous solution but in two separated containers, one for a solid form for the active agents and the other for an aqueous solution. The final aqueous solution is freshly prepared by solving the active agents in the final solution. Thereupon the final aqueous solution is filled into the nebulization chamber of the mesh nebulizer. These two containers can be completely separated containers e.g., two vials, or e.g., a dual-chamber vial. For solving the active agent e.g., a membrane between the two chambers is perforated to allow for mixing of the content of both chambers.

Thus, the present application discloses also a kit, comprising a mesh nebulizer, a first pharmaceutically acceptable container with water for injection or physiological saline solution and a second pharmaceutically acceptable container with an effective dosage of solid forms of a pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof or a pharmaceutical composition according to the invention for the prophylaxis or treatment of a coronaviral infection, wherein optionally at least one pharmaceutically acceptable excipient is contained in the first pharmaceutically acceptable container and/or the second pharmaceutically acceptable container.

The aerosol generated by the method according to the invention is administered, respectively self-administered by means of a mouthpiece. Optionally, such a mouthpiece can be additionally included in the beforementioned kits.

A common way to transfer the provided aqueous solution or final aqueous solution into the nebulization chamber of the mesh nebulizer by means of a syringe equipped with an injection needle. First, the aqueous solution is drawn up into the syringe and then injected into the nebulization chamber. Optionally, such a syringe and/or injection needle can be additionally included in the beforementioned kits. Without being limiting, typical syringes made of polyethylene, polypropylene or cyclic olefin co-polymers can be used, and a typical gauge for a stainless-steel injection needle would be in the range of 14 to 27.

In yet another aspect of the invention a pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof or a pharmaceutical composition according to the invention for use in the prophylaxis or treatment of a coronaviral infection is disclosed, wherein said pharmaceutical combination or pharmaceutical composition is provided as an additive to the ventilation air of a cardiopulmonary bypass device, a form of assisted ventilation. When the patients' conditions in intensive care unit deteriorate, they often need to be ventilated in such a device for an indefinite period of time until their own respiration would allow for a sufficient oxygen supply. Good results have been achieved when with an aerosol in a metered-dose inhaler combined with an inhalation chamber at the Y-piece. This can increase the applied dosage of bronchodilators by the factor 1.5 to 4 (Fuller et al. (1994) Chest 105: 214-218). 38% of the pharmaceutically active agent could be delivered (Marik et al. (1999) Chest 115: 1653-1657). Alternatively, a constant output mesh nebulizer yielded rates of 10 - 15%, as assessed in a scintigraphic study (Dugernier et al. (2016) Ann Intensive Care 6: 73). Vibrating mesh nebulizers delivered better results than ultrasonic or jet nebulizers for administration of antibiotics. When a constant output vibrating-mesh nebulizer is placed on the inspiratory limb at 10 cm of the Y-piece and specific ventilation parameters (tidal volume of 8 ml/kg, respiratory rate of 12 c/min, duty cycle of 50%, constant and low inspiratory flow rate inferior to 30 l/min and end inspiratory pause of 20%) are set, 63% of the administered drug (ceftazidime, amikacin) reach the inlet of the endotracheal tube, versus 37% extrapulmonary deposition (Lu et al. (2011) Am J Respir Crit Care Med 184: 106-115). Mostly, the administered drug is evenly distributed between both lungs. In pigs, the use of helium (He/O₂) instead of nitrogen (N₂/O₂) in inhaled gas was found to increase ceftazidime concentrations in subpleural lung specimens (Tonnelier et al. (2005) Anesthesiology 102: 995-1000).

In these cases, a pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof can be added to the intubated ventilation air in solid form (dry powder) or in liquid form (in an aqueous solution or as a nebulized aerosol, as described before).

The present application discloses thus likewise a pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof or a pharmaceutical composition according to the invention for use in the prophylaxis or treatment of a coronaviral infection, wherein said substance, composition or combination is added to the ventilation air of a cardiopulmonary bypass device.

In yet another aspect of the invention a pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof or a pharmaceutical composition according to the invention for use in the prophylaxis or treatment of a coronaviral infection, wherein said pharmaceutical combination or pharmaceutical composition is applied in form of liposomes, micelles, multilamellar vesicles or a cyclodextrin complex.

In yet another aspect of the invention the present application relates to a pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof or a pharmaceutical composition according to the invention for use in the prophylaxis or treatment of a coronaviral infection in a formulation for sublingual tablets.

In yet another aspect of the invention the present application relates to a pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof or a pharmaceutical composition according to the invention for use in the prophylaxis or treatment of a coronaviral infection in a liquid dosage form.

The present application discloses also the parenteral administration of a pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof or a pharmaceutical composition according to the invention in the prophylaxis or treatment of a coronaviral infection in the form of intravenous injection, intraarterial injection or intraperitoneal injection.

These liquid dosage forms comprise solutions, suspensions and emulsions. Examples are water and water/propylene glycol solutions for parenteral injections, or the addition of a sweetener or opacifier for oral solutions, suspensions and emulsions.

These liquid dosage forms can be stored in vials, IV bags, ampoules, cartridges, and prefilled syringes. Suitable excipients include solubilizers, stabilizers, buffers, tonicity modifiers, bulking agents, viscosity enhancers/reducers, surfactants, chelating agents, and adjuvants.

In yet another aspect of the invention a pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof or a pharmaceutical composition according to the invention for use in the prophylaxis or treatment of a coronaviral infection, wherein said pharmaceutical combination or pharmaceutical composition is formulated as a lyophilizate. A lyophilizate can be reconstituted with water for injection or physiological saline or a water/ethanol solution and then be administered by injection.

Typical application forms for intravenous injections include infusion pumps, hypodermic needles, drip chambers, peripheral cannulas (peripheral venous catheters) and pressure bags.

In general, an aqueous solution or a physiological saline solution is preferred. In case of a poorly soluble pharmaceutical agent according to the invention also ethanol or ethanol/water mixtures can be used.

Further suitable liquid dosage forms include drops, eyedrops and eardrops.

While SARS-CoV and MERS-CoV infect above all the lower airways SARS-CoV-2 infects first the pharynx/throat area. Only a minor percentage of these patients develops later a pulmonary infection and a pneumonia. While these pharyngeal infections cause usually only mild symptoms as in a cold or no symptoms at all these patients are highly infectious for their environment. In most cases they are unaware that they have become spreaders of the infection. Therefore, there is a medical need to treat coronaviral infections already when they are still in the pharyngeal stage, not only for treating such a patient but also for epidemiologic reasons to prevent the spreading of the epidemic. For patients with a pharyngeal infection only a systemic route of administration e.g., intravenously or perorally, with a highly effective drug or drug combination that may also cause adverse side effects is not ideal. Thus, it is desirable to provide routes of administration that treat the infected pharyngeal tissue locally.

Therefore, in yet another aspect of the invention the present application relates to a pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof or a pharmaceutical composition according to the invention for use in the prophylaxis or treatment of a coronaviral infection in a formulation for pharyngeal administration.

Administration of a medication to the pharynx can be carried out by topical administrations, such as brushing of the throat/pharynx area with a suitable liquid dosage form as drops, a lotion or a tincture, or with a viscous dosage form such as a gel or hydrogel, gurgling with a mouthwash, a sublingual tablet, a lozenge, a throat spray or a posterior pharyngeal wall injection.

A lotion is a low-viscosity topical preparation intended for application to the skin or the mucosa. Lotions are applied to the skin or mucosa with bare hands, a brush, a clean cloth, or cotton wool.

An advantage of a lotion is that it may be spread thinly and may cover a large area of skin or mucosa. Typical drugs that can be administered in form of a lotion include antibiotics, antiseptics, antifungals, corticosteroids, anti-acne agents, soothing, smoothing, moisturizing or protective agents, or anti-allergens.

Most lotions are oil-in-water emulsions using a substance such as cetearyl alcohol to keep the emulsion together, but water-in-oil lotions are also formulated. The key components are the aqueous and oily phases, an emulgent to prevent separation of these two phases and the drug substance(s). A wide variety of excipients such as fragrances, glycerol, petroleum jelly, dyes, preservatives, proteins and stabilizing agents are commonly added to lotions.

Thickness, consistency and viscosity of the lotion can be adjusted during manufacturing. Manufacturing lotions can be carried out in two cycles: a) Emollients and lubricants are dispersed in oil with blending and thickening agents. b) Perfume, color and preservatives are dispersed in the water phase. Pharmaceutically active principles are broken up in both cycles depending on the raw materials involved and the desired properties of the lotion.

A tincture is typically an alcoholic extract or formulation. Solvent concentrations of 25-60% (or even 90%) are common. Other solvents for producing tinctures include vinegar, glycerin, diethyl ether and propylene glycol. Ethanol has the advantage of being an excellent solvent for both acidic and alkaline constituents. A tincture using glycerin is called a glycerite. Glycerin is generally a poorer solvent than ethanol. Vinegar, being acidic, is a better solvent for obtaining alkaloids but a poorer solvent for acidic components.

A gel is a colloid in which the solid disperse phase forms a network in combination with that of the fluid continuous phase, resulting in a viscous semirigid sol. Gel properties range from soft and weak to hard and tough. They are defined as a substantially dilute cross-linked system, which exhibits no flow in the steady-state. By weight, gels are mostly liquid, yet they behave like solids due to a three-dimensional cross-linked network within the liquid. It is the crosslinking within the fluid that gives a gel its consistency and contributes to the adhesive stick. Gels are a dispersion of molecules of a liquid within a solid medium.

A hydrogel is a network of polymer chains that are hydrophilic, sometimes found as a colloidal gel in which water is the dispersion medium. From the hydrophilic polymer chains being held together by cross-links results a three-dimensional solid. Because of the inherent cross-links, the structural integrity of the hydrogel network does not dissolve from the high concentration of water. Hydrogels are highly absorbent (they can contain over 90% water) natural or synthetic polymeric networks. Hydrogels also possess a degree of flexibility very similar to natural tissue, due to their significant water content. In medicine, hydrogels can encapsulate chemical systems which upon stimulation by external factors such as a change of pH may cause specific pharmaceutically active agent(s) to be liberated to the environment, in most cases by a gel-sol transition to the liquid state.

Suitable gel formers can be selected from the group comprising, but not limited to, agar, algin, alginic acid, bentonite, carbomer, carrageenan, hectorite, hydroxyethyl cellulose, hydroxypropyl cellulose, polyvinyl alcohol, polyvinyl pyrrolidone, sodium carbomer.

A mouthwash is a liquid which is held in the mouth passively or swilled around the mouth by contraction of the perioral muscles and/or movement of the head, and may be gargled, where the head is tilted back and the liquid bubbled at the back of the mouth. An aqueous or alcoholic solution of a pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof or a pharmaceutical composition according to the invention can thus be formulated and administered to the pharynx.

Sublingual drug delivery can be an alternative when compared to oral drug delivery as sublingually administered dosage forms bypass hepatic metabolism. A rapid onset of pharmacological effect is often desired for some drugs, especially those used in the treatment of acute disorders. Sublingual tablets disintegrate rapidly, and the small amount of saliva present is usually sufficient for achieving disintegration of the dosage form coupled with better dissolution and increased bioavailability.

The drug must be lipophilic enough to be able to partition through the lipid bilayer, but not so lipophilic such that once it is in the lipid bilayer, it will not partition out again. According to the diffusive model of absorption, the flux across the lipid bilayer is directly proportional to the concentration gradient. Therefore, lower salivary solubility results in lower absorption rates and vice versa. In general, a drug which has been formulated for sublingual should ideally have a molecular weight of less than 500 to facilitate its diffusion. The oral cavity has a narrow pH range which lies between 5.0 to 7.0. The inclusion of a suitable buffer during the formulation of an ionizable drug makes it possible to control the pH of aqueous saliva.

For avoiding a possibly unpleasant taste or smell of the drug taste masking is needed. Sweeteners, flavors, and other taste-masking agents are essential components. Sugar-based excipients quickly dissolve in saliva and produce endothermic heat of dissolution. They create a pleasant feeling in the mouth and are most suitable for sublingual tablets along with other flavors.

Typical techniques for manufacturing sublingual tablets include direct compression, compression molding, freeze drying and hot melt extrusion (Khan et al. (2017) J Pharmaceut Res 16: 257-267).

When swallowing is avoided, an administration of a pharmaceutically active agent by means of a sublingual tablet can also reach the pharynx/throat topically. Absorption of the pharmaceutically active agent occurs to a good part via the pharyngeal mucosa.

A lozenge (troche) is a small disc-shaped or rhombic body composed of solidifying paste containing an astringent, antiseptic, or demulcent drug, used for local treatment of the mouth or throat, the lozenge being held in the mouth until dissolved. The vehicle or base of the loze nge is usually sugar, made adhesive by admixture with acacia or tragacanth, fruit paste, made from black or red currants, confection of rose, or balsam of tolu.

In particular, the present application relates to a pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof or a pharmaceutical composition according to the invention for use in the prophylaxis or treatment of a coronaviral infection in a formulation for pharyngeal administration, wherein the pharyngeal administration is carried out by means of a throat spray.

A throat spray is a medicated liquid administered to the throat as a spray, typically for the treatment of a sore throat or cough.

A throat spray may typically contain a local anesthetic (e.g., lidocaine, benzocaine), an antiseptic (e.g., chlorhexidine, cetylpyridinium chloride), herbal extracts or a combination thereof. Whatever the formulation, it should not contain too much sugar or ethanol, which further irritates the mucosa. And finally, the user should not experience any unpleasant aftertaste.

The standard for throat sprays is currently a metering pump attached to a bottle containing between 10 to 30 ml of a liquid formulation. The formulation is filled into a glass or plastic bottle with the pump fixed by a screw closure, crimped on or simply snapped onto the bottle neck. Irrespective of the fixing option selected, the system should be tight, with no leakage observed during carrying or handling by the user. Usually, the container is made from glass or plastic.

Typically, a throat spray pump will deliver a dose in the range of 50 to 200 µl per actuation. For a targeted administration, the pump will be equipped with an actuator with a prolonged nozzle. The nozzle length may range from 30 to 70 mm. It is easier to target the affected area with such a long-fixed nozzle, but this can be too bulky for users to carry, which is why actuators with foldable or swivel-mounted nozzles are preferred.

Alternatively, devices utilize continuous valves. A continuous valve delivers a targeted treatment but not precise dosing, as the formulation will be aerosolized while the actuator is pressed down. One technical solution is a tin or aluminum can with pressurized head space. When actuating the valve, the elevated internal pressure will force the formulation out of the can as long as the valve stem is pressed down.

A related but more sophisticated system is the bag-on-valve (BOV) system. The product is placed inside a bag while a propellant (in most cases compressed air) is filled in the space between the bag and the outer can. The product is squeezed out of the bag by the compressed air when the continuous valve is actuated. A BOV system will work with any 360° orientation.

Care should be taken, as throat spray formulations may contain ingredients that are very aggressive and can lower the surface tension. A simple test for spray performance will ensure that the formulation can be aerosolized by the system and that the delivered spray pattern and particle size is appropriate for the intended use.

Spray pattern and droplet size distribution are the most important parameters for a throat spray. Spray pattern is a term used to describe the spray angle and the shape of the plume for a fully developed spray. The droplet size is characterized once the spray is fully developed using a laser diffraction method. Fine particles (droplets with less than 10 µm mean dynamic diameter) should be as low as possible to avoid droplet deposition in the lower airways.

Recently, some carragelose-based throat sprays emerged, claiming protection to virus born upper respiratory infections. The first polymer of this platform is Carragelose^{®}, a broadly active anti-viral compound for treating respiratory diseases. The compound prevents the binding of viruses on the mucosal cells, in addition to its moistening effect.

Alternatively, a portable nebulizer with a high output rate and a tuned droplet size for deposition in the upper airways can be used. Breathing through a face mask can deposit droplets on the mucosa of the whole upper airways (cf. Marx and Nadler (2018) Drug Development & Delivery*).*

In particular, the present application relates to a pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof or a pharmaceutical composition according to the invention for use in the prophylaxis or treatment of a coronaviral infection in a formulation for pharyngeal administration, wherein the pharyngeal administration is carried out by means of a posterior pharyngeal wall injection.

This technique is used for pharyngoplasty by injection of calcium hydroxyapatite and other methods in plastic surgery. However, also a local injection can be made into the pharyngeal tissue for administering a pharmaceutically active agent. The injection solution can be roughly the same as for intravenous or intramuscular injections. Preferred are aqueous solutions, physiological saline solutions or, in case of a rather lipophilic pharmaceutically active agent, an ethanol/water mixture.

In a further aspect of the invention the present application relates to a pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof or a pharmaceutical composition according to the invention for use in the prophylaxis or treatment of a SARS-CoV-2 infection in a formulation for nasal administration.

In particular, the nasal administration is carried out by means of a nasal spray or nose drops.

The common formulation types used for nasal spray products are solutions, suspensions, and emulsions. Nasal spray formulations may be aqueous, hydroalcoholic, or nonaqueous-based. Depending on the type of system, the formulation will include a range of functional excipients, including solvents and cosolvents; mucoadhesive agents; pH buffers; antioxidants; preservatives; osmolality and tonicity agents; penetration enhancers; suspending agents; and surfactants. The choice of formulation type and the excipients selected will be driven by the solubility and stability of the pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof, as well as the concentration needed to deliver an efficacious dose in a typical 100 µl spray (cf. Kulkarni and Shaw (2016) in: Essential Chemistry for Formulators of Semisolid and Liquid Dosages, Elsevier). The aforementioned Carragelose^{®} technique is used also for nasal sprays.

Nose drops are administered in a similar formulation but dropwise instead of a push on the dispenser.

In particular, the present application relates to a pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof or a pharmaceutical composition according to the invention for use in a formulation in the prophylaxis or treatment of a SARS-CoV-2 infection for nasal administration, wherein the nasal administration is carried out by means of a nasal spray or nose drops.

It is known that the eye mucosae are another entry point of SARS-CoV-2 to the organism e.g., a person carries the viruses on his hands while rubbing his eyes.

Therefore, the present application relates also to a pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof or a pharmaceutical composition according to the invention, wherein the administration of said pharmaceutical combination or pharmaceutical composition according to the invention is carried out by means of eye drops.

Eye drops are mostly aqueous solutions containing a pharmaceutically active agent. The pH is usually adjusted to 7.1 to 7.5. Common buffers for eye drops are boric acid and monobasic sodium phosphate. The tonicity should be adjusted by 0.9 % saline (or another isotonizing agent such as potassium nitrate, boric acid, sodium acetate, sodium acetate phosphate buffer or mannitol) to an osmotic pressure isotonic to the cornea epithelium (225 - 430 mosm/kg). Suitable preservatives include thiomersal, organic mercury compounds such as phenylmercury, benzalkonium chloride, chlorhexidine and benzylic alcohol. For prolonging the contact time viscosity-increasing substances (thickening agents) such as cellulose derivatives (hypromellose, methylcellulose, hydroxypropyl methylcellulose), hyaluronic acid, cellulose acetate phthalate, polyethylene glycol, polyvinyl alcohols or poloxamers can be added. Wetting agents or surfactants such as benzalkonium chloride, polysorbate 20, polysorbate 80, dioctyl sodium sulphosuccinate can be included. Some amino acids, alone or in combination with sodium hyaluronate may be helpful in promoting tissue reconstitution, if needed. Suitable amino acids are glycine, leucine, lysine and proline (cf. EP 1940381 B1).

Surprisingly, it can be shown that the concomitant administration of a pharmaceutical combination according to the invention not only shows a general supraadditive effect, but also shows this effect over the whole great range of fixed ratios tested.

The term "ratio" or "fixed ratio" hereby refers to any kind of ratio between two components independent of the units used. Such a ratio is valid for weight, weight %, concentration specifications and any other feasible unit in the field of pharmaceuticals. Hence, for example a ratio of the 6'-methoxycinchonan-9-ol or a pharmaceutically acceptable salt thereof to 5-amino-2,3-dihydro-1,4-phthalazinedione or one of its pharmacologically acceptable salts of 1:10 could for example be implemented as 1mg:10mg, 1mg/kg:10mg/kg, 1mM/10mM or 1%:10% or in any other unit, as long as the ratio itself is 1:10.

The present patent application also refers to a pharmaceutical combination comprising of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof or a pharmaceutical composition according to the invention for use in the prophylaxis or treatment of a coronaviral infection, wherein the efficacy of the prophylaxis or treatment is significantly improved compared to the respective treatment with quinine or quinidine alone.

Further, the pharmaceutical combination according to the disclosure can thus be used in the prophylaxis or treatment of a coronaviral infection, wherein the components can be used in any ratio.

However, to receive the best effects the medical indication to be treated, the potency of the pharmaceutical combination according to the disclosure, the application form and feasibility of the ratio should be considered from a practical point of view, the latter particularly for maintaining patient compliance.

In the following some possible combinations and respective ratios are provided, however, the pharmaceutical combinations according to the invention are not limited to these examples. Depending on application form, indication to be treated and patients personal risk situation quinine is administered for example as tablets of 2 x 324 mg q8hr (every 8 hours) in the treatment of malaria or nocturnal leg cramps. When used as an injection solution a loading dose of 20 mg/kg quinine is recommended, followed by 10 mg/kg q8hr.

For anti-arrhythmic treatment 200 mg quinidine sulfate p.o. are given as a test dose, respectively 2 mg/kg BW (body weight). Full dosages are 30 mg/kg BW per day or 900mg/m²/per day are recommended, alternatively 15-60mg/kg BW per day divided in 5 doses q6hr. For the treatment of atrial fibrillation 300-400 mg p.o. q6hr are recommended. For the treatment of PSVT (paroxysmal supraventricular tachycardia) 400-600 mg p.o. q2-3hr are recommended. For the treatment of atrial/ventricular premature contractions 200-300 mg p.o. q6-8hr are recommended. The maintenance dose is 200-400 mg p.o. q6-8hr. The daily dose should not exceed 3-4 g/day. The recommendations for quinidine gluconate are correspondingly higher.

5-amino-2,3-dihydro-1,4-phthalazinedione sodium salt has been shown to be very safe, however, due to compliance reasons dosages should not exceed 10 g/d in case of e.g., tablets or capsules.

The pharmaceutical combination according to the disclosure can thus be used in the prophylaxis or treatment of a coronaviral infection, wherein quinine or a pharmaceutically acceptable salt thereof and 5-amino-2,3-dihydro-1,4-phthalazinedione or one of its pharmaceutically acceptable salts, hydrates and solvates can be preferably used in any ratio from 4:1 to 1:50, more preferably from 3:1 to 1:25, still more preferably 2:1 to 1:10, most preferably from 1:1 to 1:3.

The pharmaceutical combination according to the disclosure can thus be used in the prophylaxis or treatment of a coronaviral infection, wherein quinidine or pharmaceutically salt thereof and 5-amino-2,3-dihydro-1,4-phthalazinedione or one of its pharmaceutically acceptable salts, hydrates and solvates can be preferably used in any ratio from 8:1 to 1:15, more preferably from 6:1 to 1:10, still more preferably 4:1 to 1:5, most preferably from 2:1 to 1:2.

The present application refers thus to a pharmaceutical combination for use according to the disclosure, wherein the weight ratio of the dosages of quinine or one of its pharmaceutically acceptable salts to 5-amino-2,3-dihydro-1,4-phthalazinedione or one of its pharmaceutically acceptable salts, hydrates or solvates is in the range of 4:1 to 1:50, and the weight ratio of the dosages of quinidine or one of its pharmaceutically acceptable salts to 5-amino-2,3-dihydro-1,4-phthalazinedione or one of its pharmaceutically acceptable salts, hydrates or solvates is in the range of 8:1 to 1:15.

However, in case of liquid applications higher doses and/or amounts could be administered without negative impact on patient's compliance.

Due to the supraadditivity of the pharmaceutical combinations of the invention the dose of quinine or quinidine or pharmaceutically salt thereof can be reduced depending on individual patient, indication and ratio of components used. In some cases, dose and ratio adaption over time might become necessary to receive best results.

Thus, the dosage of quinine or a pharmaceutically acceptable salt thereof in the pharmaceutical combination of the invention can be reduced to 80%, preferably 50%, most preferably 20% compared to the dosage when quinine or a pharmaceutically acceptable salt thereof is administered alone.

Thus, the dosage of quinidine or pharmaceutically salt thereof in the pharmaceutical combination of the invention can be reduced to 80%, preferably 50%, most preferably 20% compared to the dosage when quinidine or pharmaceutically salt thereof is administered alone.

In a further aspect of the invention a method of treatment of a coronaviral infection is disclosed, in which an effective dose of a pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof or a pharmaceutical composition according to the invention is administered to a patient in need thereof or to a healthy person in risk of being infected with a coronavirus.

The present disclosure refers likewise to 5-amino-2,3-dihydro-1,4-phthalazinedione or one of its pharmaceutically acceptable salts, hydrates or solvates for use in a method for the treatment and/or prevention of a coronaviral infection in a subject, wherein the method comprises administering to the subject an affective amount of 5-amino-2,3-dihydro-1,4-phthalazinedione or one of its pharmaceutically acceptable salts, hydrates or solvates and concomitantly or subsequently an effective amount of a 6'-methoxycinchonan-9-ol or a pharmaceutically acceptable salt thereof.

The present disclosure refers likewise to 5-amino-2,3-dihydro-1,4-phthalazinedione or one of its pharmaceutically acceptable salts, hydrates or solvates for use in a method for the treatment and/or prevention of a coronaviral infection in a subject, wherein the method comprises administering to the subject an affective amount of 5-amino-2,3-dihydro-1,4-phthalazinedione or one of its pharmaceutically acceptable salts, hydrates or solvates and concomitantly or subsequently an effective amount of a 6'-methoxycinchonan-9-ol or a pharmaceutically acceptable salt thereof, wherein 5-amino-2,3-dihydro-1,4-phthalazinedione is a sodium salt.

The present disclosure refers likewise to 5-amino-2,3-dihydro-1,4-phthalazinedione or one of its pharmaceutically acceptable salts, hydrates or solvates for use in a method for the treatment and/or prevention of a coronaviral infection in a subject, wherein the method comprises administering to the subject an affective amount of a 6'-methoxycinchonan-9-ol or a pharmaceutically acceptable salt thereof and concomitantly or subsequently an effective amount of 5-amino-2,3-dihydro-1,4-phthalazinedione or one of its pharmaceutically acceptable salts, hydrates or solvates.

The present disclosure refers likewise to 5-amino-2,3-dihydro-1,4-phthalazinedione or one of its pharmaceutically acceptable salts, hydrates or solvates for use in a method for the treatment and/or prevention of a coronaviral infection in a subject, wherein the method comprises administering to the subject an affective amount of a 6'-methoxycinchonan-9-ol or a pharmaceutically acceptable salt thereof and concomitantly or subsequently an effective amount of 5-amino-2,3-dihydro-1,4-phthalazinedione or one of its pharmaceutically acceptable salts, hydrates or solvates, wherein 5-amino-2,3-dihydro-1,4-phthalazinedione is a sodium salt.

### EXAMPLES

In all experiments, solutions containing 5-amino-2,3-dihydro-1,4-phthalazinedione sodium salt are prepared using the beforementioned anhydrous Form I of 5-amino-2,3-dihydro-1,4-phthalazinedione sodium salt (provided by MetrioPharm). Quinine sulfate and quinidine sulfate are purchased from Buchler GmbH, Braunschweig, Germany.

Example 1: A pharmaceutical combination of 5-amino-2,3-dihydro-1,4-phthalazinedione sodium salt and quinine inhibits the replication of SARS-CoV-2 in infected Calu-3 cells

In order to investigate whether a pharmaceutical combination of 5-amino-2,3-dihydro-1,4-phthalazinedione sodium salt and remdesivir has an effect on the spread of viral infection, Western Blot (WB) analyses are carried out. Calu-3 cells (cf. Park et al. (2021) Biomol Ther 29: 273-281) were received from Prof. Jan Munch, Institute of Molecular Virology, University of Ulm, Germany. They are maintained in Minimal Essential Medium (MEM) containing 20% (v/v) inactivated FCS, 1 mM I-glutamine, 100 U/mL penicillin, and 100 µg/mL streptomycin and 1 mM sodium pyruvate. Confluent monolayers of Calu-3 cells are infected for one hour in FCS-free DMEM with a 100-fold dilution of the field isolate SARS-CoV-2_{PR-1} (isolated from a 61 years old patient six days after the presumed date of infection and two days after start of mild COVID-19 symptoms). In parallel experiments cells are then washed with PBS (phosphate buffer saline), provided with fresh medium containing 5-amino-2,3-dihydro-1,4-phthalazinedione sodium salt alone, quinine alone or a pharmaceutical combination of 5-amino-2,3-dihydro-1,4-phthalazinedione sodium salt and quinine in non-cytotoxic concentrations, respectively. The treatment with a pharmaceutical combination of 5-amino-2,3-dihydro-1,4-phthalazinedione sodium salt and quinine is carried out over the entire experimental procedure. 3 days post infection (dpi) virus-containing cell culture supernatants are harvested. Virions are purified from cell culture supernatants via a 20% (w/v) sucrose cushion (20,000 × g, 4°C, 90 min). Cells are washed with PBS and the pellet is dissolved in SDS (sodium dodecyl sulfate) sample buffer, separated by SDS-PAGE gel electrophoresis, transferred onto nitrocellulose membranes and blocked with 3% bovine serum albumin, and incubated with the appropriate primary antibody against SARS-CoV-2 nucleocapsid. SARS-CoV-2 protein is visualized using a convalescent SARS-CoV-2 patient serum. The antihuman secondary antibody coupled to horseradish peroxidase is obtained from Dianova (Hamburg, Germany). Visualization is effected by means of an electrochemiluminescence reaction.

Herein, for all three groups an inhibition of SARS-Cov-2 replication is shown in Calu-3 cells by a reduction of SARS-CoV-2 nucleocapsid protein.

Densitometric evaluations of SARS-CoV-2 nucleocapsid are carried out with the analysis program AIDA^{®}. Densitometric evaluation allows for the quantification of signal intensities in Western Blot and thus for conclusions on the quantity of a certain protein in the sample. The evaluation clearly shows that after the addition of 5-amino-2,3-dihydro-1,4-phthalazinedione sodium salt alone, quinine alone or a pharmaceutical combination of 5-amino-2,3-dihydro-1,4-phthalazinedione sodium salt and quinine the generation of SARS-CoV-2 proteins is inhibited dose-dependently. The much stronger inhibition by the pharmaceutical combination suggests a supraadditive effect. Statistical analyses are performed using unpaired T Test with Welch's correction; **p<0.01, *p<0.05.

### Example 2: A pharmaceutical combination of 5-amino-2,3-dihydro-1,4-phthalazinedione sodium salt and quinine inhibits the replication of SARS-CoV-2 in infected Calu-3 cells in a dose-dependent manner in qRT-PCR

To evaluate these results qRT-PCR (quantitative real-time polymerase chain reaction) experiments are conducted, a very sensitive method to determine quantitatively the number of SARS-CoV-2 RNA copies in infective virions released from cells in the supernatant. The method was performed according to Corman et al. (2020) Euro Surveill 25: 2000045. Calu-3 cells (see Example 1) are infected with SARS-CoV2 for 1 h. Cells are then washed with PBS, provided with fresh medium containing 5-amino-2,3-dihydro-1,4-phthalazinedione sodium salt alone, quinine alone or a pharmaceutical combination of 5-amino-2,3-dihydro-1,4-phthalazinedione sodium salt and quinine in non-cytotoxic concentration ranges, respectively. The treatment is carried out over the entire experimental procedure. 3 days post infection (dpi) the virus-containing supernatants are harvested. Samples containing the released virions are quantified by real-time PCR AgPath-ID One-Step RT-PCR Kit from Ambion (Cat: 4387424), allowing reverse transcription, cDNA synthesis and PCR amplification in a single step. Samples are analyzed by 7500 software v2.3 (Applied Bioscience).

Using this very sensitive method a dose-dependent reduction in the amount of SARS-Cov-2 RNA copies can be shown. This method confirms the results from Example 1. The reduction by combinations of 5-amino-2,3-dihydro-1,4-phthalazinedione sodium salt and quinine is again significantly higher than after treatment with 5-amino-2,3-dihydro-1,4-phthalazinedione sodium salt alone or quinine alone.

### Example 3: In effective concentrations 5-amino-2,3-dihydro-1,4-phthalazinedione sodium salt alone, quinine alone or the pharmaceutical combination of 5-amino-2,3-dihydro-1,4-phthalazinedione sodium salt and quinine are not cytotoxic in Calu-3 cell cultures

For addressing the question whether 5-amino-2,3-dihydro-1,4-phthalazinedione sodium salt alone, quinine alone or the pharmaceutical combination of 5-amino-2,3-dihydro-1,4-phthalazinedione sodium salt and quinine show a cytotoxic effect in the abovementioned systems non-infected Calu-3 cells (see Example 1) are treated in parallel to the Western blot studies with increasing concentrations of 5-amino-2,3-dihydro-1,4-phthalazinedione sodium salt (0.25 mM, 0.5 mM, 1 mM, 2 mM, 4 mM), quinine or the pharmaceutical combination of 5-amino-2,3-dihydro-1,4-phthalazinedione sodium salt and quinine. Toxicity is assessed with a WST (water-soluble tetrazolium salt 1) assay. Herein viable cells with an intact mitochondrial succinate-tetrazolium dehydrogenase system effect an enzymatic conversion of the feebly red tetrazolium salt WST-1 (4-[3-(4-iodophenyl)-2-(4-nitrophenyl)-2*H*-5-tetrazolio]-1,3 benzene disulfonate) into dark red formazan. This color change can be measured photometrically in a spectrophotometer. Thus, the WST assay is a very sensitive method for measuring the toxicity of substances on the cell metabolism. The value for untreated cells is set to 100%.

It can be shown that none of 5-amino-2,3-dihydro-1,4-phthalazinedione sodium salt, quinine or the pharmaceutical combination of 5-amino-2,3-dihydro-1,4-phthalazinedione sodium salt and quinine displays any significant toxic effect in antivirally effective concentrations in Calu-3 cells during an observation period of 3 days.

1 µM staurosporine (an indolocarbazole compound from *Streptomyces staurosporeus,* an apoptosis inducer) is used as positive control. Statistical analyses are performed using unpaired T Test with Welch's correction; **p<0.01, *p<0.05.

Thus, it can be stated that the antiviral effects of 5-amino-2,3-dihydro-1,4-phthalazinedione sodium salt alone, quinine alone or the pharmaceutical combination of 5-amino-2,3-dihydro-1,4-phthalazinedione sodium salt and quinine are not due to unspecific cytotoxic effects.

### Example 4: A pharmaceutical combination of 5-amino-2,3-dihydro-1,4-phthalazinedione sodium salt and quinidine inhibits the replication of SARS-CoV-2 in infected Calu-3 cells

The experiment is carried analogous to Example 1, using different concentrations of quinidine alone or in combination with 5-amino-2,3-dihydro-1,4-phthalazinedione sodium salt. For 5-amino-2,3-dihydro-1,4-phthalazinedione sodium salt alone the results from Example 1 are taken.

It shows that quinidine alone or in combination with 5-amino-2,3-dihydro-1,4-phthalazinedione sodium salt reduce dose-dependently the replication of SARS-CoV-2 at non-cytotoxic concentrations. The results for the pharmaceutical combination suggest a supraadditive effect.

### Example 5: A pharmaceutical combination of 5-amino-2,3-dihydro-1,4-phthalazinedione sodium salt and quinidine inhibits the replication of SARS-CoV-2 in infected Calu-3 cells in a dose-dependent manner in qRT-PCR

The experiment is carried analogous to Example 2, using different concentrations of quinidine alone or in combination with 5-amino-2,3-dihydro-1,4-phthalazinedione sodium salt.

For 5-amino-2,3-dihydro-1,4-phthalazinedione sodium salt alone the results from Example 2 are taken.

These experiments confirm that quinidine alone or in combination with 5-amino-2,3-dihydro-1,4-phthalazinedione sodium salt reduce dose-dependently the replication of SARS-CoV-2 at non-cytotoxic concentrations. The results for the pharmaceutical combination suggest a supraadditive effect.

### Example 6: In effective concentrations 5-amino-2,3-dihydro-1,4-phthalazinedione sodium salt alone, quinidine alone or the pharmaceutical combination of 5-amino-2,3-dihydro-1,4-phthalazinedione sodium salt and quinidine are not cytotoxic in Calu-3 cell cultures

The experiment is carried analogous to Example 3, using different concentrations of quinidine alone or in combination with 5-amino-2,3-dihydro-1,4-phthalazinedione sodium salt. For 5-amino-2,3-dihydro-1,4-phthalazinedione sodium salt alone the results from Example 3 are taken.

It can be shown that none of 5-amino-2,3-dihydro-1,4-phthalazinedione sodium salt, quinidine or the pharmaceutical combination of 5-amino-2,3-dihydro-1,4-phthalazinedione sodium salt and quinidine displays any significant toxic effect in antivirally effective concentrations in Calu-3 cells during an observation period of 3 days.

Thus, it can be stated that the antiviral effects of 5-amino-2,3-dihydro-1,4-phthalazinedione sodium salt alone, quinidine alone or the pharmaceutical combination of 5-amino-2,3-dihydro-1,4-phthalazinedione sodium salt and quinidine are not due to unspecific cytotoxic effects.

## Claims

1. Pharmaceutical combination of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof.

2. Pharmaceutical combination according to claim 1 for use in medicine.

3. Pharmaceutical combination according to claim 1 or 2 for use in the prophylaxis or treatment of a coronaviral infection.

4. Pharmaceutical combination for use according to any one of claims 1 to 3, wherein the pharmaceutically acceptable salt of 5-amino-2,3-dihydro-1,4-phthalazinedione is 5-amino-2,3-dihydro-1,4-phthalazinedione sodium salt.

5. Pharmaceutical combination for use according to any one of claims 1 to 4, wherein 5-amino-2,3-dihydro-1,4-phthalazinedione sodium salt is provided as one of crystalline anhydrate polymorph forms I, II or III **characterized by** crystallography values determined by means of x-ray powder diagrams:
d values: 13.5; 6.9; 5.2; 4.6; 3.9; 3.5; 3.4; 3.3; 3.1; 3.0 and/or
2-theta values: 6.5; 12.7; 16.9; 19.3; 22.8; 25.8; 26.6; 27.2; 28.7; 30.3 for Form I, d values: 12.9; 7.9; 7.1; 6.5; 5.3; 4.0; 3.7; 3.6; 3.3; 3.2 and/or
2-theta values: 6.8; 11.2; 12.5; 13.7; 16.7; 22.4; 24.3; 24.9; 27.2; 27.8 for Form II, and
d values: 13.131; 7.987; 7.186; 6.566; 6.512; 5.372; 3.994; 3.662; 3.406; 3.288; 3.283; 3.222; 3.215; 3.127; 2.889 and/or
2-theta values: 6.73; 11.07; 12.31; 13.48; 13.59; 16.49; 22.24; 24.29; 26.14; 27.10; 27.14; 27.67; 27.72; 28.52; 30.93 for Form III.

6. Pharmaceutical combination for use according to any one of claims 1 to 5, wherein said coronaviral infection is selected from a group consisting of SARS-CoV, MERS-CoV, SARS-CoV-2, HCoV-HKU1, HCoV-NL-63, HCoV-OC43 and HCoV-229E infections.

7. Pharmaceutical combination for use according to any one of claims 1 to 6, wherein the weight ratio of the dosages of quinine or one of its pharmaceutically acceptable salts to 5-amino-2,3-dihydro-1,4-phthalazinedione or one of its pharmaceutically acceptable salts, hydrates or solvates is in the range of 4:1 to 1:50, and the weight ratio of the dosages of quinidine or one of its pharmaceutically acceptable salts to 5-amino-2,3-dihydro-1,4-phthalazinedione or one of its pharmaceutically acceptable salts, hydrates or solvates is in the range of 8:1 to 1:15.

8. Pharmaceutical composition for use in the prophylaxis or treatment of a coronaviral infection, wherein said composition contains of 5-amino-2,3-dihydro-1,4-phthalazinedione or one of its pharmaceutically acceptable salts, hydrates or solvates and a 6'-methoxycinchonan-9-ol or a pharmaceutically acceptable salt thereof, a carrier and at least one pharmaceutically acceptable excipient.

9. Pharmaceutical composition for use according to claim 8, wherein said pharmaceutical composition is applied orally in the form of tablets, soft gelatin capsules, hard gelatin capsules, sugar-coated tablets, pills, powders, granulates, juices, syrups, drops, teas, solutions or suspensions in aqueous or non-aqueous liquids, edible foams, mousses, oil-in-water emulsions or water-in-oil emulsions.

10. Pharmaceutical composition according to claim 8 for use in the prophylaxis or treatment of a coronaviral infection in a formulation for inhalatory administration.

11. Pharmaceutical composition according to claim 8 for use in the prophylaxis or treatment of a coronaviral infection, wherein said substance, composition or combination is added to the ventilation air of a cardiopulmonary bypass device.

12. Pharmaceutical composition according to claim 8 for use in the prophylaxis or treatment of a coronaviral infection in a formulation for sublingual tablets.

13. Pharmaceutical composition according to claim 8 for use in the prophylaxis or treatment of a coronaviral infection, wherein the administration of said pharmaceutical composition is carried out by means of a nose spray, nose drops or eye drops.

14. Pharmaceutical composition according to claim 8 for use in the prophylaxis or treatment of a coronaviral infection in a formulation for pharyngeal administration.

15. A method of treatment of a coronaviral infection in which an effective dose of a pharmaceutical composition as defined in Claim 7 is administered to a patient in need thereof or to a healthy person at risk of being infected with a coronavirus.
